# EUROPÄISCHE PATENTSCHRIFT

(11) **EP 2 182 027 B1**
(45) Veröffentlichungstag und Bekanntmachung des Hinweises auf die Patenterteilung: **28.03.2012**
(21) Anmeldenummer: 09174410.2
(22) Anmeldetag: 29.10.2009
(51) Int. Cl.: C08L 5/00, C08L 39/06, C08L 33/14

(54) **Neues Verdickungssystem**
New thickening system
Nouveau système d'épaississement

(30) Priorität: 30.10.2008 DE 102008053883
(43) Veröffentlichungstag der Anmeldung: 05.05.2010
(73) Patentinhaber: Henkel AG & Co. KGaA, 40589 Düsseldorf (DE)
(72) Erfinder: Struwe, Alexandra, 47877, Willich (DE); Waldmann-Laue, Marianne, 40789, Monheim (DE); Stipps, Fredrik, 42719, Solingen (DE)

(56) Entgegenhaltungen:
- EP-A- 2 033 617
- WO-A-2004/000248
- DE-A1- 10 327 348

## Beschreibung

Gegenstand der Erfindung sind verdickte Zusammensetzungen, die ein Verdickersystem enthaltend Dehydroxanthan Gum und mindestens ein vernetztes Copolymer aus Vinylpyrrolidon und 2-Methyl-2[(1-oxo-2-propenyl)amino]-1-propansulfonsäure, sowie mindestens einen polymeren Verdicker in Form eines inversen, auto-inversiblen Polymerlatex, sowie der Verwendung der erfindungsgemäßen Verdickerkombination zur Verdickung wässriger Mittel, insbesondere von Zusammensetzungen mit einem pH-Wert kleiner pH 6.

Im Stand der Technik sind viele Verdicker und Verdickerkombinationen beschrieben. Dennoch bleibt die Verdickung wässriger Systeme, insbesondere bei sauren pH-Werten und/oder hohen Elektrolyconzentrationen, eine formulierungstechnische Herausforderung. Die Rheologie solcher verdickten Zusammensetzungen ist mit Blick auf die Effektivität der Verdickung und auf die Stabilität der Verdickungswirkung noch verbesserungswürdig.

Aufgabe der vorliegenden Erfindung ist es daher, ein Verdickungssystem bereitzustellen, das auch bei niedrigen pH-Werten effektiv und langzeitstabil eingesetzt werden kann.

Die Anmelderin hat nun überraschenderweise gefunden, dass sich wässrige Zusammensetzungen - auch solche mit niedrigen pH-Werten - hervorragend durch ein Verdickersystem, enthaltend Dehydroxanthan Gum und mindestens ein vernetztes Copolymer aus Vinylpyrrolidon und 2-Methyl-2[(1-oxo-2-propenyl)amino]-1-propansulfonsäure, sowie mindestens einen polymeren Verdicker in Form eines inversen, auto-inversiblen Polymerlatex, verdicken lassen. Die Verdickung ist effektiv und über einen langen Zeitraum stabil. Die erzielte Verdickung verleiht den Zusammensetzungen bei der Anwendung unter anderem eine leichte Handhabbarkeit und erhöht den Verbleib des Mittels bei Applikation an vorbestimmten Orten.

Ein erster Gegenstand der Erfindung ist daher eine verdickte Zusammensetzung, enthaltend
a) 20 bis 95 Gew.-% Wasser und
b) 0,05 bis 5 Gew.-% Dehydroxanthan Gum und
c) mindestens ein vernetztes Copolymer aus Vinylpyrrolidon und 2-Methyl-2[(1-oxo-2-propenyl)amino]-1-propansulfonsaure, das teilweise oder vollständig neutralisiert ist,
d) mindestens einen polymeren Verdicker In Form eines Inversen, auto-inversiblen Polymerlatex, enthaltend eine Olphase, eine Wasserphase, mindestens einen Öl-in-Wasser-Emulgator und mindestens einen verzweigten oder vernetzten Polyelektrolyten, wobei der Polyelektrolyt ausgewählt ist aus
   i) einem Homopolymer, dessen Monomer eine schwache Säurefunktion enthält, die teilweise oder vollständig neutralisiert ist,
   ii) einem Copolymer, das mindestens ein Monomer mit einer starken Säurefunktion sowie ein neutrales Monomer oder ein Monomer mit einer schwachen Säurefunktion enthält.

Soweit nicht anders erwähnt, beziehen sich Mengenangaben im Rahmen der vorliegenden Anmeldung immer auf die gesamte Zusammensetzung, physikalische Größen werden bei 20°C und 1013,25 mbar gemessen.

Das Verdickersystem eignet sich hervorragend zur Verdickung wässrig-basierter Systeme, so dass die erfindungsgemäßen Zusammensetzungen - bezogen auf Ihr Gewicht, - 20 bis 95 Gew.-% Wasser enthalten. Bevorzugte erfindungsgemäße Zusammensetzungen sind dadurch gekennzeichnet, dass sie, bezogen auf ihr Gewicht, 30 bis 94 Gew.-%, vorzugsweise 35 bis 92,5 Gew.-%, besonders bevorzugt 40 bis 90 Gew.-%, weiter bevorzugt 50 bis 87,5 Gew.-% und insbesondere 60 bis 85 Gew.-% Wasser enthalten.

Als ersten Bestandteil der Verdickerkombination enthalten die erfindungsgemäßen Zusammensetzungen 0,05 - 5 Gew.-% Dehydroxanthan Gum (dehydratisiertes Xanthan-Gum mit der INCI-Bezeichnung Dehydroxanthan Gum). Die Herstellung von dehydratisiertem Xanthan-Gum ist z.B. aus JP 07278202 A bekannt. Dehydratisiertes Xanthan-Gum mit der INCl-Bezeichnung Dehydroxanthan Gum ist unter dem Handelsnamen Amaze^{®} XT von National Starch/Akzo Nobel erhältlich. Von National Starch sind auch zahlreiche Formulierungsbeispiele mit Amaze^{®} XT veröffentlicht, denen der Fachmann jedoch keinen Hinweis auf eine besondere Eignung und besondere Vorteile des Rohstoffs Amaze^{®} XT zur Verdickung saurer Zusammensetzungen entnehmen kann.

Erfindungsgemäß besonders bevorzugte Zusammensetzungen sind dadurch gekennzeichnet, dass sie, bezogen auf ihr Gewicht, 0,1 - 4 Gew.-%, besonders bevorzugt 0,25 - 3 Gew.-%, weiter bevorzugt 0,5 - 2,5 Gew.-% und insbesondere 1 - 2 Gew.-% Dehydroxanthan Gum b) enthalten.

Das in den erfindungsgemäßen Zusammensetzungen als zweiter Bestandteil der Verdickerkombination enthaltene vernetzte Copolymer aus Vinylpyrrolidon und 2-Methyl-2[(1-oxo-2-propenyl)-amino]-1-propansulfonsäure, das teilweise oder vollständig neutralisiert ist, ist erhältlich durch Copolymerisation dieser beiden Monomere in Gegenwart von Ammoniumhydroxid und einem Vernetzungsmittel. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Bevorzugte erfindungsgemäße Zusammensetzungen enthalten bezogen auf ihr Gewicht, 0,05 - 3 Gew.-%, vorzugsweise 0,1 - 2 Gew.-%, besonders bevorzugt 0,2 - 1,5 Gew.-%, weiter bevorzugt 0,28 - 1,25 Gew.-% und insbesondere 0,5 bi-s 1 Gew.-% mindestens eines vernetzten Copolymers c) aus Vinylpyrrolidon und 2-Methyl-2[(1-oxo-2-propenyl)amino]-1-propansulfonsäure, das teilweise oder vollständig neutralisiert ist.

Als dritten Bestandteil der Verdickerkombination enthalten die erfindungsgemäßen Zusammensetzungen mindestens einen polymeren Verdicker d) in Form eines inversen, auto-inversiblen Polymerlatex, enthaltend eine Ölphase, eine Wasserphase, mindestens einen Öl-in-Wasser-Emulgator und mindestens einen verzweigten oder vernetzten Polyelektrolyten, der ein Copolymer darstellt, das mindestens ein Monomer mit einer starken Säurefunktion sowie mindestens ein weiteres Monomer aufweist, das neutral ist oder eine schwache Säurefunktion enthält.

Besonders bevorzugte erfindungsgemäße Zusammensetzungen sind dadurch gekennzeichnet, dass sie, bezogen auf ihr Gewicht, 0,1 - 5 Gew.-%, vorzugsweise 0,25 - 4 Gew.-%, besonders bevorzugt 0,5 - 3 Gew.-%, weiter bevorzugt 0,75 - 2,5 Gew.-% und insbesondere 1 - 2 Gew.-% mindestens eines polymeren Verdickers d) in Form eines inversen, auto-inversiblen Polymerlatex enthalten.

Bevorzugte Polyelektrolyten sind aus zwei, drei, vier, fünf oder sechs verschiedenen Monomeren aufgebaut, von denen mindestens ein Monomer eine starke Säurefunktion aufweist und das mindestens eine weitere Monomer neutral ist oder eine schwache Säurefunktion aufweist.

Bevorzugt sind die Monomerkombinationen
starke Säurefunktion/neutral,
starke Säurefunktion/schwache Säurefunktion,
starke Säurefunktion/schwache Säurefunktion/neutral
starke Säurefunktion/schwache Säurefunktion 1/schwache Säurefunktion 2/neutral,
starke Säurefunktion/schwache Säurefunktion/neutral 1/neutral 2,
starke Säurefunktion/schwache Säurefunktion 1/schwache Säurefunktion 2/neutral 1/neutral 2. Die oben angegebene Reihenfolge der Auflistung der Monomerbausteine gibt dabei nicht notwendigerweise die tatsächliche Reihenfolge der Monomerbausteine im Polyelektrolytmolekül wieder.

In einer bevorzugten Zusammensetzung ist die schwache Säurefunktion des Polyelektrolytmonomeren im polymeren Verdicker d) eine Carboxylgruppe -COOH, die teilweise oder vollständig neutralisiert ist.

Aus der partiellen Neutralisation der schwachen Säurefunktion resultieren der neutralisierte Monomerbaustein und der nicht-neutralisierte, saure Monomerbaustein, beispielsweise Acrylsäure und Natriumacrylat. Beide Monomere werden im Sinne der vorliegenden Anmeldung als gleich angesehen. Dementsprechend werden beide Monomere im Sinne der vorliegenden Anmeldung als "Monomer mit schwacher Säurefunktion" angesehen.

Besonders bevorzugte Beispiele für solche schwach sauren Polyelektrolytmonomeren sind Acrylsäure, Methacrylsäure, Itaconsäure und Maleinsäure. Bevorzugte erfindungsgemäße Zusammensetzungen sind daher dadurch gekennzeichnet, dass die schwache Säurefunktion des Polyelektrolytmonomeren eine Carboxylgruppe -COOH darstellt, die teilweise oder vollständig neutralisiert ist, wobei weiter bevorzugte erfindungsgemäße Zusammensetzungen dadurch gekennzeichnet sind, dass die Monomere des Polyelektrolyten, die mit der schwach sauren -COOH-Gruppe funktionalisiert sind, ausgewählt sind aus Acrylsäure, Methacrylsäure, Itaconsäure und Maleinsäure, die teilweise oder vollständig neutralisiert sind.

In einer weiteren bevorzugten Zusammensetzungen ist die starke Säurefunktion des Polyelektrolytmonomeren im polymeren Verdicker d) ausgewählt aus einer Sulfonsäuregruppe -SO₃H und einer Phosphonsäuregruppe, die teilweise oder vollständig neutralisiert sind.

Ein besonders bevorzugtes Polyelektrolytmonomer mit starker Säurefunktion ist 2-Methyl-2[(1-oxo-2-propenyl)amino]-1-propansulfonsäure, die teilweise oder vollständig neutralisiert ist, so dass bevorzugte erfindungsgemäße Zusammensetzungen dadurch gekennzeichnet sind, dass das Polyelektrolytmonomer mit starker Säurefunktion im polymeren Verdicker d) ausgewählt ist aus 2-Methyl-2[(1-oxo-2-propenyl)amino]-1-propansulfonsäure, die teilweise oder vollständig neutralisiert ist.

Aus der partiellen Neutralisation der starken Säurefunktion resultieren der neutralisierte Monomerbaustein und der nicht-neutralisierte, saure Monomerbaustein, beispielsweise 2-Methyl-2[(1-oxo-2-propenyl)amino]-1-propansulfonsäure und Natrium-2-methyl-2[(1-oxo-2-propenyl)amino]-1-propansulfonat. Beide Monomere werden im Sinne der vorliegenden Anmeldung als gleich angesehen. Dementsprechend werden beide Monomere im Sinne der vorliegenden Anmeldung als "Monomer mit starker Säurefunktion" angesehen.

Bevorzugte erfindungsgemäße Zusammensetzungen sind daher dadurch gekennzeichnet, dass die starke Säurefunktion des Polyelektrolytmonomeren ausgewählt ist aus einer Sulfonsäuregruppe -SO₃H und einer Phosphonsäuregruppe, die teilweise oder vollständig neutralisiert sind, wobei weiter bevorzugte erfindungsgemäße Zusammensetzungen dadurch gekennzeichnet sind, dass das Polyelektrolytmonomer mit starker Säurefunktion ausgewählt ist aus 2-Methyl-2[(1-oxo-2-propenyl)amino]-1-propansulfonsäure, die teilweise oder vollständig neutralisiert ist.

Im Sinne der vorliegenden Erfindung sind alle Polyelektrolytmonomere, die mit einer schwachen oder starken Säuregruppe funktionalisiert sind, teilweise oder vollständig neutralisiert als Natrium-, Kalium-, Ammonium-, Ethanolamin- oder Aminosäure-Salz. Bevorzugte erfindungsgemäße Zusammensetzungen sind daher dadurch gekennzeichnet, dass die Polyelektrolytmonomere im polymeren Verdicker d), die mit einer schwachen oder starken Säuregruppe funktionalisiert sind, teilweise oder vollständig neutralisiert sind als Natrium-, Kalium-, Ammonium-, Ethanolamin- oder Aminosäure-Salz.

In einer weiteren bevorzugten erfindungsgemäßen Zusammensetzung ist das neutrale Polyelektrolytmonomer im polymeren Verdicker d) ausgewählt aus 2-Hydroxyethylacrylat, 2,3-Dihydroxypropylacrylat, 2-Hydroxyethylmethacrylat, 2,3-Dihydroxypropylmethacrylat, den ethoxylierten Derivaten der vorstehend genannten Ester mit einem Molekulargewicht zwischen 400 und 1000 g/mol, Acrylamid, Alkylacrylamiden, wie beispielsweise Methylacrylamid, Ethylacrylamid, n-Propylacrylamid und Isopropylacrylamid, Dialkylamiden, wie beispielsweise Dimethylacrylamid, Diethylacrylamid, Di-n-propylacrylamid und Diisopropylacrylamid, sowie Polyvinylpyrrolidon. In bevorzugten erfindungsgemäßen Zusammensetzungen ist das neutrale Polyelektrolytmonomer ausgewählt aus 2-Hydroxyethylacrylat, 2,3-Dihydroxypropylacrylat, 2-Hydroxyethylmethacrylat, 2,3-Dihydroxypropylmethacrylat, den ethoxylierten Derivaten der genannten Ester mit einem Molekulargewicht zwischen 400 und 1000 g/mol, Acrylamid, Dimethylacrylamid, Diethylacrylamid, Di-n-propylacrylamid, Diisopropylacrylamid sowie Polyvinylpyrrolidon. Besonders bevorzugte neutrale Polyelektrolytmonomere sind ausgewählt aus 2-Hydroxyethylacrylat, Acrylamid, Dimethylacrylamid und Polyvinylpyrrolidon.

Vernetzte Copolymere aus 2-Methyl-2[(1-oxo-2-propenyl)amino]-1-propansulfonsäure (AMPS) und Hydroxyethylacrylat, in Form eines inversen, auto-inversiblen Latex sind kommerziell erhältlich, z. B. unter den Handelsnamen Simulgel^{®}NS, Simulgel^{®}I-NS 100, Simulgel^{®}FL und Sepiplus^{®} S von der Firma Seppic.

Weiterhin besonders bevorzugt sind teilweise oder vollständig neutralisierte, vernetzte Copolymere aus 2-Methyl-2[(1-oxo-2-propenyl)amino]-1-propansulfonsäure (AMPS) und Acrylamid, in Form eines inversen, auto-inversiblen Latex kommerziell erhältlich, z. B. unter dem Handelsnamen Simulgel^{®}-600 von der Firma Seppic.

Weiterhin besonders bevorzugt sind teilweise oder vollständig neutralisierte, vernetzte Copolymere aus 2-Methyl-2[(1-oxo-2-propenyl)amino]-1-propansulfonsäure (AMPS) und Acrylsäure, in Form eines inversen, auto-inversiblen Latex kommerziell erhältlich, z. B. unter den Handelsnamen Simulgel^{®}EG und Simulgel^{®}EPG von der Firma Seppic.

Weiterhin besonders bevorzugt sind teilweise oder vollständig neutralisierte, vernetzte Copolymere aus 2-Methyl-2[(1-oxo-2-propenyl)amino]-1-propansulfonsäure (AMPS), Acrylsäure bzw. Natriumacrylat, und (als drittem Monomerbaustein) Dimethylacrylamid, in Form eines inversen, auto-inversiblen Latex kommerziell erhältlich, z. B. unter dem Handelsnamen Simulgel^{®}SMS 88 von der Firma Seppic.

Allen vorgenannten, bevorzugt verwendeten inversen Polymerlatices ist gemein, dass sie mindestens ein Öl, bevorzugt ausgewählt aus weißen Mineralölen, Squalan, Isohexadecan und (gegebenenfalls hydriertem) Polyisobuten, sowie mindestens einen Öl-in-Wasser-Emulgator, ausgewählt aus den Ethylenoxid-Addukten von Sorbitanoleat, Ricinusöl, das gewünschtenfalls gehärtet ist, Sorbitanlaurat und Laurylalkohol, sowie weiterhin ausgewählt aus der Polyethylenoxid-freien Öl-in-Wasser-Emulgatorklasse der C6-C22-Fettalkohol-Glucoseether, insbesondere Caprinic Glucoside, Caprylic Glucoside, Capric Glucoside, Lauric Glucoside, Myristic Glucoside, Cetyl Glucoside, Stearyl Glucoside, Arachidyl Glucoside, Behenyl Glucoside, besonders bevorzugt Caprylic Glucoside und Capric Glucoside, enthalten. Demnach sind bevorzugte erfindungsgemäße Zusammensetzungen dadurch gekennzeichnet, dass die Ölphase des inversen Polymerlatex mindestens ein Öl, ausgewählt aus weißen Mineralölen, Squalan, Isohexadecan und (gegebenenfalls hydriertem) Polyisobuten, enthält. Weiter bevorzugte erfindungsgemäße Zusammensetzungen sind dadurch gekennzeichnet, dass der in dem inversen Polymerlatex enthaltene Öl-in-Wasser-Emulgator ausgewählt ist aus den Ethylenoxid-Addukten von Sorbitanoleat, Ricinusöl, das gewünschtenfalls gehärtet ist, Sorbitanlaurat und Laurylalkohol, aus der Polyethylenoxid-freien Öl-in-Wasser-Emulgatorklasse der C6-C22-Fettalkohol-Glucoseether, insbesondere Caprinic Glucoside, Caprylic Glucoside, Capric Glucoside, Lauric Glucoside, Myristic Glucoside, Cetyl Glucoside, Stearyl Glucoside, Arachidyl Glucoside, Behenyl Glucoside, besonders bevorzugt Caprylic Glucoside und Capric Glucoside.

Die erfindungsgemäß bevorzugten verdickenden Polymerlatices d) weisen vorzugsweise einen Polymergehalt von 30 - 90 Gew.-%, bevorzugt 35 - 75 Gew.-% und besonders bevorzugt 40 - 60 Gew.-%, jeweils bezogen auf den gesamten Latex, auf. Der Polymergehalt des Latex hat dabei jedoch eher nur eine Bedeutung für die Herstellung der erfindungsgemäßen Zusammensetzungen, z. B. in Bezug auf die Mischbarkeit bzw. Dosierbarkeit. Für die erfindungsgemäßen Zusammensetzungen selbst ist eher der Polymergehalt an sich, bezogen auf die erfindungsgemäße Zusammensetzung, bedeutsam.

Wird die erfindungsgemäße verdickte Zusammensetzung als Kosmetikum formuliert, wird - bezogen auf die gesamte erfindungsgemäße Zusammensetzung - das verdickende Polymer d) vorzugsweise in Mengen von 0,1 - 5 Gew.-%, bevorzugt 0,3 - 3 Gew.-% und besonders bevorzugt 0,5 - 2,5 Gew.-%, bezogen auf die gesamte erfindungsgemäße kosmetische Zusammensetzung, eingesetzt, wobei Polymergehalte von 0,4, 0,6, 0,7, 0,8, 0,9, 1,0, 1,1, 1,2, 1,3, 1,4, 1,5, 1,6, 1,7, 1,8, 1,9, 2,0, 2,1, 2,2, 2,3 und 2,4 Gew.-% besonders bevorzugt und Polymergehalte von 0,3, 0,4, 0,5, 0,6, 0,7, 0,8, 0,9, 1,0 und 1,1 Gew.-% außerordentlich bevorzugt sein können.

Wie bereits erwähnt, eignet sich das erfindungsgemäße Verdickersystem besonders hervorragend zur Verdickung saurer wässriger Zubereitungen. Erfindungsgemäß besonders bevorzugte Zusammensetzungen sind daher dadurch gekennzeichnet, dass sie einen pH-Wert von 1 bis 6,9, vorzugsweise 2 bis 6,5, besonders bevorzugt 2,5 bis 6, außerordentlich bevorzugt 3 bis 5,5, weiter bevorzugt 3,5 bis 5,0, weiter bevorzugt 4,0 bis 4,5 aufweisen.

Saure wässrige Zubereitungen können in einer Vielzahl von Anwendungsgebieten eingesetzt werden, z.B. im Rahmen der Metallbehandlung und -vorbehandlung, der Konsumgüter usw. Besonders bevorzugte erfindungsgemäße Zusammensetzungen sind Waschmittel, Waschhilfsmittel, Reinigungsmittel für harte Oberflächen, Geschirrspülmittel oder kosmetische Zubereitungen. Ganz besonders bevorzugt eignen sich die erfindungsgemäßen Verdickungssysteme für WC-Reiniger, Wäscheweichspülmittel und Kosmetika.

Eine erfindungsgemäß besonders bevorzugte Zusammensetzung ist dadurch gekennzeichnet, dass sie als Toilettenreinigungsmittel konfektioniert ist, das zusätzlich mindestens eine organische Säure, ausgewählt aus Citronensäure, Ameisensäure, Essigsäure, Milchsäure, Amidosulfonsäure, Weinsäure, Glycolsäure, Maleinsäure und ihren Mischungen, enthält.

Als kalklösende Mittel sind in solchen Toilettenreinigungsmitteln vorzugsweise kalklösende Säuren wie die Citronensäure, Ameisensäure, Essigsäure, Milchsäure, Amidosulfonsäure, Weinsäure, Glycolsäure, Maleinsäure oder deren wasserlöslichen Salze enthalten, die vorzugsweise in einer Menge - bezogen auf die Mittel - von 1 - 12, vorzugsweise 2 - 7 Gew.-% eingesetzt werden können. Daneben können auch anorganische Säuren wie Salzsäure oder Phosphorsäure enthalten sein.

Weitere übliche Inhaltsstoffe von WC-Reinigern, die in den erfindungsgemäßen Zusammensetzungen dieser Ausführungsform enthalten sein können, sind Tenside, Lösemittel, Lösungsvermittler, Komplexbildner, Parfums, Farbstoffe, Gerüststoffe, Konservierungsmittel, antibakterielle Substanzen usw.. Besonders bevorzugte erfindungsgemäße Mittel dieser Ausführungsform enthalten mindestens einen Inhaltsstoff aus der Gruppe der Aniontenside, der nichtionischen Tenside, der Farbstoffe und der Duftstoffe.

Eine weitere erfindungsgemäß besonders bevorzugte Zusammensetzung ist dadurch gekennzeichnet, dass sie als Wäscheweichspülmittel konfektioniert ist, das zusätzlich mindestens ein kationisches Tensid und/oder einen textilweichmachenden Ton enthält.

Solche Wäscheweichspülmittel dienen der "Konditionierung" oder der avivierenden Behandlung von Textilien, Stoffen und Geweben. Durch die Konditionierung werden den Textilien positive Eigenschaften verliehen, wie beispielsweise ein verbesserter Weichgriff, eine erhöhte Glanz- und Farbbrillanz, ein verbesserter Dufteindruck, Verringerung der Filzbildung, Bügelerleichterung durch Verringerung der Gleiteigenschaften, Verringerung des Knitterverhaltens und der statischen Aufladung sowie eine Farbübertragungsinhibierung bei gefärbten Textilien.

Beispiele für kationische Tenside sind quartäre Ammoniumverbindungen, kationische Polymere und Emulgatoren, wie sie in Haarpflegemitteln und auch in Mitteln zur Textilavivage eingesetzt werden. Geeignete Beispiele sind quartäre Ammoniumverbindungen der Formeln (III) und (IV), 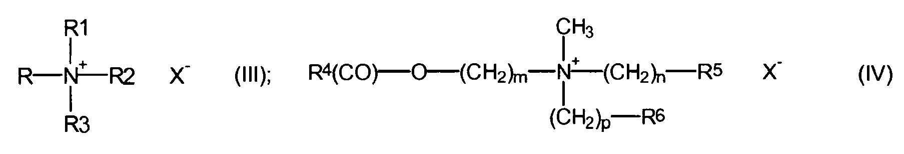 wobei in (III) R und R¹ für einen acyclischen Alkylrest mit 12 bis 24 Kohlenstoffatomen, R² für einen gesättigten C₁-C₄ Alkyl- oder Hydroxyalkylrest steht, R³ entweder gleich R, R¹ oder R² ist oder für einen aromatischen Rest steht. X⁻ steht entweder für ein Halogenid-, Methosulfat-, Methophosphat- oder Phosphation sowie Mischungen aus diesen. Beispiele für kationische Verbindungen der Formel (III) sind Didecyldimethylammoniumchlorid, Ditalgdimethylammoniumchlorid oder Dihexadecylammoniumchlorid.

Besonders bevorzugt sind Mittel, die zusätzlich gewebeweichmachende Komponenten auf Basis von Esterquats aufweisen. Verbindungen der Formel (IV), (V) und (VII) sind so genannte Esterquats. Esterquats zeichnen sich durch eine hervorragende biologische Abbaubarkeit aus. In Formel (IV) steht R⁴ für einen aliphatischen Alkylrest mit 12 bis 22 Kohlenstoffatomen mit 0, 1, 2 oder 3 Doppelbindungen; R⁵ steht für H, OH oder O(CO)R⁷, R⁶ steht unabhängig von R⁵ für H, OH oder O(CO)R⁸, wobei R⁷ und R⁸ unabhängig voneinander jeweils für einen aliphatischen Alk(en)ylrest mit 12 bis 22 Kohlenstoffatomen mit 0, 1, 2 oder 3 Doppelbindungen steht. m, n und p können jeweils unabhängig voneinander den Wert 1, 2 oder 3 haben. X⁻ kann entweder ein Halogenid-, Methosulfat-, Methophosphat- oder Phosphation sowie Mischungen aus diesen sein. Bevorzugt sind Verbindungen, die für R⁵ die Gruppe O(CO)R⁷ und für R⁴ und R⁷ Alkylreste mit 16 bis 18 Kohlenstoffatomen enthalten. Besonders bevorzugt sind Verbindungen, bei denen R⁶ zudem für OH steht. Beispiele für Verbindungen der Formel (IV) sind Methyl-N-(2-hydroxyethyl)-N,N-di(talgacyl-oxyethyl)ammonium-methosulfat, Bis-(palmitoyl)-ethyl-hydroxyethyl-methyl-ammonium-methosulfat oder Methyl-N,N-bis(acyloxyethyl)-N-(2-hydroxyethyl)ammonium-methosulfat. Werden quarternierte Verbindungen der Formel (IV) eingesetzt, die ungesättigte Alkylketten aufweisen, sind die Acylgruppen bevorzugt, deren korrespondierenden Fettsäuren eine Jodzahl zwischen 5 und 80, vorzugsweise zwischen 10 und 60 und insbesondere zwischen 15 und 45 aufweisen und die ein cis/trans-Isomerenverhältnis (in Gew.-%) von größer als 30 : 70, vorzugsweise größer als 50 : 50 und insbesondere größer als 70 : 30 haben. Handelsübliche Beispiele sind die von Stepan unter dem Warenzeichen Stepantex^{®} vertriebenen Methylhydroxyalkyldialkoyloxyalkylammoniummethosulfate oder die unter Dehyquart^{®} bekannten Produkte von Cognis bzw. die unter Rewoquat^{®} bekannten Produkte von Goldschmidt-Witco. Weitere bevorzugte Verbindungen sind die Diesterquats der Formel (V), die unter dem Namen Rewoquat® W 222 LM bzw. CR 3099 erhältlich sind und neben der Weichheit auch für Stabilität und Farbschutz sorgen. 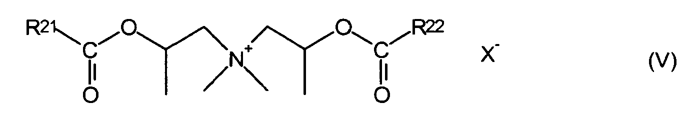 R²¹ und R²² stehen dabei unabhängig voneinander jeweils für einen aliphatischen Rest mit 12 bis 22 Kohlenstoffatomen mit 0, 1, 2 oder 3 Doppelbindungen.

Neben den oben beschriebenen quartären Verbindungen können auch andere bekannte Verbindungen eingesetzt werden, wie beispielsweise quartäre Imidazoliniumverbindungen der Formel (VI), 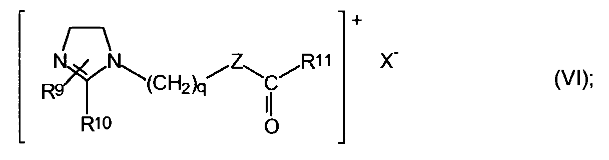 wobei R⁹ für H oder einen gesättigten Alkylrest mit 1 bis 4 Kohlenstoffatomen, R¹⁰ und R¹¹ unabhängig voneinander jeweils für einen aliphatischen, gesättigten oder ungesättigten Alkylrest mit 12 bis 18 Kohlenstoffatomen, R¹⁰ alternativ auch für O(CO)R²⁰ stehen kann, wobei R²⁰ einen aliphatischen, gesättigten oder ungesättigten Alkylrest mit 12 bis 18 Kohlenstoffatomen bedeutet, und Z eine NH-Gruppe oder Sauerstoff bedeutet und X⁻ ein Anion ist. q kann ganzzahlige Werte zwischen 1 und 4 annehmen.

Weitere besonders bevorzugte Esterquats sind durch Formel (VII) beschrieben, 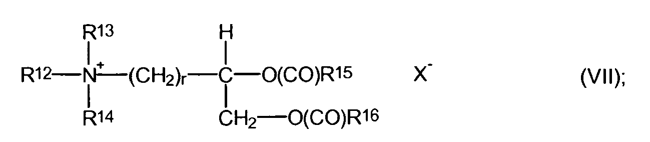 wobei R¹², R¹³ und R¹⁴ unabhängig voneinander für eine C₁₋₄-Alkyl-, Alkenyl- oder Hydroxyalkylgruppe steht, R¹⁵ und R¹⁶ jeweils unabhängig ausgewählt eine C₈₋₂₈-Alkylgruppe darstellt und r eine Zahl zwischen 0 und 5 ist.

Neben den Verbindungen der Formeln (III) und (IV) können auch kurzkettige, wasserlösliche, quartäre Ammoniumverbindungen eingesetzt werden, wie Trihydroxyethylmethylammoniummethosulfat oder die Alkyltrimethylammoniumchloride, Dialkyldimethylammoniumchloride und Trialkylmethylammoniumchloride, z.B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid.

Auch protonierte Alkylaminverbindungen, die weichmachende Wirkung aufweisen, sowie die nicht quaternierten, protonierten Vorstufen der kationischen Emulgatoren sind geeignet. Weitere erfindungsgemäß verwendbare kationische Verbindungen stellen die quaternisierten Proteinhydrolysate dar.

Zu den geeigneten kationischen Polymeren zählen die Polyquaternium-Polymere, wie sie im CTFA Cosmetic Ingredient Dictionary (The Cosmetic, Toiletry und Fragrance Association, Inc., 1997), insbesondere die auch als Merquats bezeichneten Polyquaternium-6-, Polyquaternium-7-, Polyquaternium-10-Polymere (Ucare Polymer IR 400; Amerchol), Polyquaternium-4-Copolymere, wie Pfropfcopolymere mit einen Cellulosegerüst und quartären Ammoniumgruppen, die über Allyldimethylammoniumchlorid gebunden sind, kationische Cellulosederivate, wie kationisches Guar, wie Guar-hydroxypropyltriammoniumchlorid, und ähnliche quaternierte Guar-Derivate (z.B. Cosmedia Guar, Hersteller: Cognis GmbH), kationische quartäre Zuckerderivate (kationische Alkylpolyglucoside), z.B. das Handelsprodukt Glucquat^{®}100, gemäß CTFA-Nomenklatur ein "Lauryl Methyl Gluceth-10 Hydroxypropyl Dimonium Chloride", Copolymere von PVP und Dimethylaminomethacrylat, Copolymere von Vinylimidazol und Vinylpyrrolidon, Aminosiliconpolymere und Copolymere.

Ebenfalls einsetzbar sind polyquaternierte Polymere (z.B. Luviquat Care von BASF) und auch kationische Biopolymere auf Chitinbasis und deren Derivate, beispielsweise Chitosane.

Ebenfalls einsetzbar sind Verbindungen der Formel (VIII), 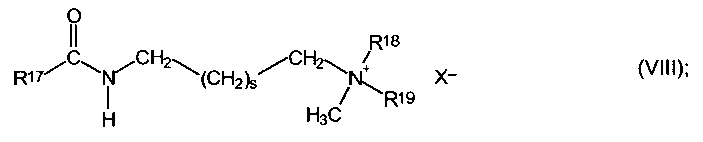 die Alkylamidoamine in ihrer nicht quaternierten oder, wie dargestellt, ihrer quaternierten Form, sein können. R¹⁷ kann ein aliphatischer Alk(en)ylrest mit 12 bis 22 Kohlenstoffatomen mit 0, 1, 2 oder 3 Doppelbindungen sein. s kann Werte zwischen 0 und 5 annehmen. R¹⁸ und R¹⁹ stehen unabhängig voneinander jeweils für H, C₁₋₄-Alkyl oder Hydroxyalkyl. Bevorzugte Verbindungen sind Fettsäureamidoamine wie das unter der Bezeichnung Tego Amid^{®}S 18 erhältliche Stearylamidopropyldimethylamin oder das unter der Bezeichnung Stepantex^{®} X 9124 erhältliche 3-Talgamidopropyl-trimethylammonium-methosulfat, die sich neben einer guten konditionierenden Wirkung auch durch farbübertragungsinhibierende Wirkung sowie speziell durch ihre gute biologische Abbaubarkeit auszeichnen.

Besonders bevorzugt sind alkylierte quaternäre Ammoniumverbindungen, von denen mindestens eine Alkylkette durch eine Estergruppe und/oder Amidogruppe unterbrochen ist, insbesondere N-Methyl-N(2-hydroxyethyl)-N,N-(ditalgacyloxyethyl)ammonium-methosulfat.

Als nichtionische Weichmacher kommen vor allem Polyoxyalkylenglycerolalkanoate, wie sie in GB 2202244, Polybutylene, wie sie in GB 2199855, langkettige Fettsäuren, wie sie in EP 13780, ethoxylierte Fettsäureethanolamide, wie sie in EP 43547, Alkylpolyglycoside, insbesondere Sorbitan- mono, -di- und triester, wie sie in EP 698140 und Fettsäureester von Polycarbonsäuren, wie sie in DE 2822891 beschrieben werden.

In dem erfindungsgemäßen Mitteln dieser Ausführungsform können Weichmacher in Mengen von 1 - 75 Gew.-%, vorzugsweise von 3 - 35 Gew-%, jeweils bezogen auf das gesamte Mittel, enthalten sein.

Ein besonders bevorzugtes Einsatzgebiet für die erfindungsgemäße Verdickerkombination stellen kosmetische Mittel dar. Kosmetische Mittel im Sinne dieser Anmeldung sind Stoffe oder Zubereitungen aus Stoffen, die dazu bestimmt sind, äußerlich am Menschen oder in seiner Mundhöhle zur Reinigung, Pflege oder zur Beeinflussung des Aussehens oder des Körpergeruchs oder zur Vermittlung von Geruchseindrücken angewendet zu werden, es sei denn, dass sie überwiegend dazu bestimmt sind, Krankheiten, Leiden, Körperschäden oder krankhafte Beschwerden zu lindern oder zu beseitigen.

Erfindungsgemäß besonders bevorzugte kosmetische Mittel sind kosmetische oder dermatologische Zusammensetzungen zur topischen Behandlung der Haut. Die erfindungsgemäße Verdickerkombination führt hier zu Produkten mit angenehmem Auftragegefühl, die sich leicht verstreichen lassen, ohne zu kleben oder zu tropfen. Mit besonderem Vorzug werden in solchen kosmetischen oder dermatologischen Zusammensetzungen zur topischen Behandlung der Haut hautberuhigende, hautpflegende, feuchtigkeitsspendende oder faltenreduzierende Wirkstoffe eingesetzt.

Es hat sich überraschenderweise gezeigt, dass sich Betain (Trimethylammoniumacetat) besonders gut in kosmetische oder dermatologische Zusammensetzungen zur topischen Behandlung der Haut einarbeiten läßt und seine Wirkung schneller und nachhaltiger entfaltet, wenn diese Mittel mit der erfindungsgemäßen Verdickerkombination viskositätsreguliert sind.

Eine weitere erfindungsgemäß besonders bevorzugte Zusammensetzung ist daher dadurch gekennzeichnet, dass sie, bezogen auf ihr Gewicht, 0,5 - 5 Gew.-%, vorzugsweise 1,0 - 4 Gew.-%, besonders bevorzugt 1,5 - 3 Gew.-% und insbesondere 1,75 - 2,5 Gew.-% Trimethylammoniumacetat (H₃C)₃N⁺CH₂COO⁻ enthält.

Ein weiterer feuchtigkeitsspendender und hautweichmachender Wirkstoff, der hervorragend mit dem erfindungsgemäßen Verdickersystem harmoniert und seine Wirkung mit diesem schneller und nachhaltiger entfaltet, ist N,N'-Bis(2-hydroxyethyl)harnstoff. Zudem bewirkt die Anwesenheit dieses Inhaltsstoffes in erfindungsgemäßen Zusammensetzungen, dass die Wirksamkeit ausgewählter kosmetischer oder dermatologischer Wirkstoffe optimiert wird.

Weitere erfindungsgemäß besonders bevorzugte Zusammensetzungen enthalten bezogen auf ihr Gewicht, 0,25 - 5 Gew.-%, vorzugsweise 0,5 - 4 Gew.-%, besonders bevorzugt 0,75 - 3 Gew.-% und insbesondere 1 - 2 Gew.-% N,N'-Bis(2-hydroxyethyl)harnstoff.

Bevorzugte optionale Inhaltsstoffe der erfindungsgemäßen kosmetischen Mittel können über ihre Funktion definiert werden. Natürlich können manche Inhaltsstoffe auch multifunktionell sein. Bevorzugte Inhaltsstoffe der erfindungsgemäßen Mittel, vorzugsweise Kosmetikprodukte können sein:
a) Antimikrobielle Stoffe
   Diese können den Produkten zugesetzt werden, um ganz allgemein die Aktivitäten von Mikroorganismen, insbesondere den Mikroorganismen, die für die Bildung von Akne verantwortlich sind, zu verringern. Zu diesen Keimen zählen unter anderem verschiedene Spezies aus der Gruppe der Staphylokokken, der Gruppe der Corynebakterien, Anaerokokken und Mikrokokken, insbesondere Corynebacterium acnes, Corynebacterium granulosum, Propionibacterium acnes, Propionibacterium avidum und Propionibacterium granulosum. Als keimhemmende oder antimikrobielle Wirkstoffe erfindungsgemäß bevorzugt sind insbesondere Organohalogenverbindungen sowie -halogenide, quartäre Ammoniumverbindungen, eine Reihe von Pflanzenextrakten und Zinkverbindungen. Weiterhin sind Phenoxyethanol, Dinatriumdihydroxyethylsulfosuccinylundecylenat, Natriumbicarbonat, Zinklactat, Natriumphenolsulfonat und Zinkphenolsulfonat, Ketoglutarsäure, Terpenalkohole wie z. B. Farnesol, Chlorophyllin-Kupfer-Komplexe, α-Monoalkylglycerinether mit einem verzweigten oder linearen gesättigten oder ungesättigten, gegebenenfalls hydroxylierten C₆ - C₂₂-Alkylrest, besonders bevorzugt α-(2-Ethylhexyl)glycerinether, im Handel erhältlich als Sensiva^{®} SC 50 (ex Schülke & Mayr), Carbonsäureester des Mono-, Di- und Triglycerins (z. B. Glycerinmonolaurat, Diglycerinmonocaprinat), Lantibiotika sowie Pflanzenextrakte (z. B. grüner Tee und Bestandteile des Lindenblütenöls) einsetzbar. Weitere bevorzugte Wirkstoffe gegen Akne und unreine Haut sind ausgewählt aus sogenannten präbiotisch wirksamen Komponenten, worunter erfindungsgemäß solche Komponenten zu verstehen sind, die nur oder zumindest überwiegend die geruchsbildenden Keime der Hautmikroflora hemmen, nicht aber die erwünschten, das heißt, die nicht-geruchsbildenden Keime, die zu einer gesunden Hautmikroflora gehören. Explizit sind hier die Wirkstoffe, die in DE 10333245 und DE 102004011968 als präbiotisch wirksam offenbart sind, mit einbezogen; dazu gehören Nadelbaumextrakte, insbesondere aus der Gruppe der Pinaceae, und Pflanzenextrakte aus der Gruppe der Sapindaceae, Araliaceae, Lamiaceae und Saxifragaceae, insbesondere Extrakte aus *Picea spp., Paullinia sp., Panax sp., Lamium album* oder *Ribes nigrum* sowie Mischungen dieser Substanzen.
b) Antiperspirantien
   Schweißhemmende Wirkstoffe haben eine porenadstringierende Wirkung und werden daher häufig auch in Hautbehandlungsmitteln zur Sebumsuppression eingesetzt. Die zur Sebumsuppression verwendeten Einsatzmengen sind dabei üblicherweise geringer als die zur Schweißhemmung verwendeten Mengen und liegen im Bereich von 0,1 - 10 Gew.-%, bevorzugt 2 - 3 Gew.-%. Bevorzugte erfindungsgemäße Mittel enthalten - zur Unterstützung der Gesamtleistung des Produktes - mindestens einen adstringierenden Antitranspirant-Wirkstoff, ausgewählt aus den wasserlöslichen adstringierenden anorganischen und organischen Salzen des Aluminiums, Zirkoniums und Zinks bzw. beliebigen Mischungen dieser Salze, enthalten ist. Erfindungsgemäß wird unter Wasserlöslichkeit eine Löslichkeit von wenigstens 5 Gew.-% bei 20 °C verstanden, das heißt, dass Mengen von wenigstens 5 g des Antitranspirant-Wirkstoffes in 95 g Wasser bei 20 °C löslich sind.
c) Chelatbildner
   Diese werden z.B. kosmetischen Mitteln zugesetzt, damit sie Komplexe mit Metallionen bilden, um so beispielsweise die Lagerstabilität der erfindungsgemäßen Mittel zu verbessern. Als komplexbildende Substanz besonders bevorzugt ist Ethylendiamintetraessigsäure (EDTA) und ihre Natriumsalze, wie sie beispielsweise unter dem Handelsnamen Trilon B von der Firma BASF erhältlich ist, weiterhin Nitrilotriessigsäure (NTA) und ihre Natriumsalze, β-Alanindiessigsäure und ihre Salze und Phosphonsäuren und deren Salze. Die mindestens eine komplexbildende Substanz ist bevorzugt in einer Gesamtmenge von 0,01 - 0,5 Gew.-%, besonders bevorzugt 0,08 - 0,2 Gew.-%, bezogen auf das Gesamtgewicht der erfindungsgemäßen Zusammensetzung, enthalten.
d) Emollientien
   Diese haben die Aufgabe, die Haut geschmeidig zu machen und zu glätten.
   Bevorzugte erfindungsgemäße Zusammensetzungen, die als Emulsion, Suspension, Spray oder Stift vorliegen, enthalten als Emollient mindestens ein bei 20 ° C flüssiges Öl, das keine Duftstoffkomponente und kein etherisches Öl darstellt. Erfindungsgemäß bevorzugte Öle sind ausgewählt aus verzweigten gesättigten oder ungesättigten Fettalkoholen mit 6 - 30 Kohlenstoffatomen. Diese Alkohole werden häufig auch als Guerbet-Alkohole bezeichnet, da sie nach der Guerbet-Reaktion erhältlich sind. Bevorzugte Alkoholöle sind Hexyldecanol (Eutanol^{®} G 16, Guerbitol^{®} T 16), Octyldodecanol (Eutanol^{®} G, Guerbitol^{®} 20) und 2-Ethylhexylalkohol. Weitere bevorzugte Ölkomponenten sind Mischungen aus Guerbetalkoholen und Guerbetalkoholestern, z.B. das Handelsprodukt Cetiol^{®} PGL (Hexyldecanol und Hexyldecyllaurat. Weitere erfindungsgemäß bevorzugte Emollient-Öle sind ausgewählt aus den Triglyceriden von linearen oder verzweigten, gesättigten oder ungesättigten, gegebenenfalls hydroxylierten C₈₋₃₀-Fettsäuren. Besonders geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maisöl, Olivenöl, Rapsöl, Sesamöl, Distelöl, Weizenkeimöl, Pfirsichkernöl und die flüssigen Anteile des Kokosöls und dergleichen sein. Geeignet sind aber auch synthetische Triglyceridöle, insbesondere Capric/Caprylic Triglycerides, sowie z.B. Glyceryltriisostearin mit verzweigten Fettsäureresten. Weitere erfindungsgemäß besonders bevorzugte Öle sind ausgewählt aus den Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat. Weitere erfindungsgemäß besonders bevorzugte Öle sind ausgewählt aus den Anlagerungsprodukten von 1 bis 5 Propylenoxid-Einheiten an ein- oder mehrwertige C₈₋₂₂-Alkanole wie Octanol, Decanol, Decandiol, Laurylalkohol, Myristylalkohol und Stearylalkohol, z. B. PPG-2-Myristylether und PPG-3-Myristylether (Witconol^{®} APM). Weitere erfindungsgemäß bevorzugte Ölkomponenten sind ausgewählt aus den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Dazu zählen Hexyldecylstearat (Eutanol^{®} G 16 S), Hexyldecyllaurat, Isodecylneopentanoat, Isononylisononanoat, 2-Ethylhexylpalmitat (Cegesoft^{®} C 24) und 2-Ethylhexylstearat (Cetiol^{®} 868). Ebenfalls bevorzugt geeignet sind Isopropylmyristat, Isopropylpalmitat, Isopropylstearat, Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Düsotridecylacetat, n-Butylstearat, n-Hexyllaurat, n-Decyloleat, Oleyloleat, Oleylerucat, Erucyloleat, Erucylerucat, Ethylenglycoldioleat und -dipalmitat. Weitere erfindungsgemäß bevorzugte Ölkomponenten sind ausgewählt aus den Anlagerungsprodukten von mindestens 6 Ethylenoxid und/oder Propylenoxid-Einheiten an ein- oder mehrwertige C₃₋₂₂-Alkanole wie Butanol, Butandiol, Myristylalkohol und Stearylalkohol, z. B. PPG-14-Butylether (Ucon Fluid^{®} AP), PPG-9-Butylether (Breox^{®} B25), PPG-10-Butandiol (Macol^{®} 57) und PPG-15-Stearylether (Arlamol^{®} E). Weitere erfindungsgemäß bevorzugte Ölkomponenten sind ausgewählt aus den C_{B}-C₂₂-Fettalkoholestern einwertiger oder mehrwertiger C₂-C₇-Hydroxycarbonsäuren, insbesondere die Ester der Glycolsäure, Milchsäure, Äpfelsäure, Weinsäure, Citronensäure und Salicylsäure. Solche Ester auf Basis von linearen C_{14/15}-Alkanolen, z. B. C₁₂-C₁₅-Alkyllactat, und von in 2-Position verzweigten C_{12/13}-Alkanolen sind unter dem Warenzeichen Cosmacol^{®} von der Firma Nordmann, Rassmann GmbH & Co, Hamburg, zu beziehen, insbesondere die Handelsprodukte Cosmacol^{®} ESI, Cosmacol^{®} EMI und Cosmacol^{®} ETI. Weitere erfindungsgemäß bevorzugte Ölkomponenten sind ausgewählt aus den symmetrischen, unsymmetrischen oder cyclischen Estern der Kohlensäure mit Fettalkoholen, z. B. Glycerincarbonat, Dicaprylylcarbonat (Cetiol^{®} CC) oder die Ester der DE 197 56 454 A1. Weitere erfindungsgemäß bevorzugte Ölkomponenten sind ausgewählt aus den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen. Es kann erfindungsgemäß außerordentlich bevorzugt sein, Mischungen der vorgenannten Öle einzusetzen. Weitere erfindungsgemäß bevorzugte Ölkomponenten sind ausgewählt aus Siliconölen und Kohlenwasserstoffölen. Besonders bevorzugte erfindungsgemäße Hautbehandlungsmittel sind dadurch gekennzeichnet, dass das/die bei 20 °C flüssige/n Öl/e in einer Gesamtmenge von 0,1 - 80 Gew.-%, bevorzugt 2 - 20 Gew.-%, besonders bevorzugt 3 - 15 Gew.-%, jeweils bezogen auf das Gesamtgewicht der Zusammensetzung, enthalten ist/sind. Neben den Ölen, das heißt, den Substanzen, die unter Normalbedingungen flüssig vorliegen, können auch bei Normalbedingungen fest vorliegende Lipid- oder Wachskomponenten als Emollient verwendet werden. Besonders bevorzugte Lipid- oder Wachskomponenten sind ausgewählt aus Kokosfettsäureglycerinmono-, -di- und -triestern, Butyrospermum Parkii (Shea Butter) und Estern von gesättigten, einwertigen C₈-C₁₈-Alkoholen mit gesättigten C₁₂-C₁₈-Monocarbonsäuren sowie Mischungen dieser Substanzen, enthalten ist. Diese niedriger schmelzenden Lipid- oder Wachskomponenten ermöglichen eine Konsistenzoptimierung des Produktes und eine Minimierung der sichtbaren Rückstände auf der Haut. Besonders bevorzugt sind Handelsprodukte mit der INCI-Bezeichnung Cocoglycerides, insbesondere die Handelsprodukte Novata^{®} (ex Cognis), besonders bevorzugt Novata^{®} AB, ein Gemisch aus C₁₂-C₁₈-Mono-, Di- und Triglyceriden, das im Bereich von 30 - 32°C schmilzt, sowie die Produkte der Softisan-Reihe (Sasol Germany GmbH) mit der INCI-Bezeichnung Hydrogenated Cocoglycerides, insbesondere Softisan 100, 133, 134, 138, 142. Weitere bevorzugte Ester von gesättigten, einwertigen C₁₂-C₁₈-Alkoholen mit gesättigten C₁₂-C₁₈-Monocarbonsäuren sind Stearyllaurat, Cetearylstearat, Cetylpalmitat und Myristylmyristat. Erfindungsgemäß bevorzugte Emollients sind weiterhin pflanzliche Wachse, z. B. Candelillawachs, Carnaubawachs, Japanwachs, Zuckerrohrwachs, Ouricourywachs, Korkwachs, Sonnenblumenwachs, Fruchtwachse wie Orangenwachse, Zitronenwachse, Grapefruitwachs, und tierische Wachse, z. B. Bienenwachs, Schellackwachs und Walrat. Im Sinne der Erfindung kann es besonders bevorzugt sein, hydrierte oder gehärtete Wachse einzusetzen. Als Wachskomponente sind auch chemisch modifizierte Wachse, insbesondere die Hartwachse, wie z. B. Montanesterwachse, hydrierte Jojobawachse und Sasolwachse, einsetzbar. Zu den synthetischen Wachsen, die ebenfalls erfindungsgemäß bevorzugt sind, zählen beispielsweise Polyalkylenwachse und Polyethylenglycolwachse, C₂₀-C₄₀-Dialkylester von Dimersäuren, C₃₀₋₅₀-Alkylbienenwachs sowie Alkyl- und Alkylarylester von Dimerfettsäuren. Eine besonders bevorzugte Wachskomponente ist ausgewählt aus mindestens einem Ester aus einem gesättigten, einwertigen C₁₆-C₆₀-Alkohol und einer gesättigten C₈-C₃₆-Monocarbonsäure. Erfindungsgemäß zählen hierzu auch Lactide, die cyclischen Doppelester von α-Hydroxycarbonsäuren der entsprechenden Kettenlänge. Ester aus Fettsäuren und langkettigen Alkoholen haben sich für die erfindungsgemäße Zusammensetzung als besonders vorteilhaft erwiesen, weil sie den erfindungsgemäßen Hautbehandlungsmitteln ausgezeichnete sensorische Eigenschaften verleihen. Die Ester setzen sich aus gesättigten verzweigten oder unverzweigten Monocarbonsäuren und gesättigten verzweigten oder unverzweigten einwertigen Alkoholen zusammen. Auch Ester aus aromatischen Carbonsäuren bzw. Hydroxycarbonsäuren (z. B. 12-Hydroxystearinsäure) und gesättigten verzweigten oder unverzweigten Alkoholen sind erfindungsgemäß einsetzbar. Besonders bevorzugt ist, die Wachskomponenten zu wählen aus der Gruppe der Ester aus gesättigten verzweigten oder unverzweigten Alkancarbonsäuren einer Kettenlänge von 12 bis 24 C-Atomen und den gesättigten verzweigten oder unverzweigten Alkoholen einer Kettenlänge von 16 bis 50 C-Atomen, die einen Schmelzpunkt > 50°C haben. Insbesondere können als Wachskomponente C₁₆₋₃₆-Alkylstearate und C₁₈₋₃₈-Alkylhydroxystearoylstearate, C₂₀₋₄₀-Alkylerucate sowie Cetearylbehenat vorteilhaft sein. Das Wachs oder die Wachskomponenten weisen einen Schmelzpunkt > 50°C, bevorzugt > 60°C, auf. Eine besonders bevorzugte Ausführungsform der Erfindung enthält als Wachskomponente ein C₂₀-C₄₀-Alkylstearat. Weitere bevorzugte Lipid- oder Wachskomponenten mit einem Schmelzpunkt > 50°C sind die Triglyceride gesättigter und gegebenenfalls hydroxylierter C₁₂₋₃₀-Fettsäuren, wie gehärtete Triglyceridfette (hydriertes Palmöl, hydriertes Kokosöl, hydriertes Rizinusöl), Glyceryltribehenat (Tribehenin) oder Glyceryltri-12-hydroxystearat, weiterhin synthetische Vollester aus Fettsäuren und Glycolen oder Polyolen mit 2 - 6 Kohlenstoffatomen, solange sie einen Schmelzpunkt oberhalb von 50 °C aufweisen, beispielsweise bevorzugt C₁₈ - C₃₆ Acid Triglyceride (Syncrowax^{®} HGL-C). Erfindungsgemäß ist als Wachskomponente hydriertes Rizinusöl, erhältlich z.B. als Handelsprodukt Cutina^{®} HR, besonders bevorzugt. Weitere bevorzugte Lipid- oder Wachskomponenten mit einem Schmelzpunkt > 50°C sind die gesättigten linearen C₁₄ - C₃₆-Carbonsäuren, insbesondere Myristinsäure, Palmitinsäure, Stearinsäure und Behensäure sowie Mischungen dieser Verbindungen, z. B. Syncrowax^{®} AW 1C (C₁₈ - C₃₆-Fettsäuren) oder Cutina^{®} FS 45 (Palmitin- und Stearinsäure).
e) Emulgatoren /Dispergatoren
   Hierbei handelt es sich um oberflächenaktive Substanzen, die vorzugsweise im Stande sind, nicht mischbare Flüssigkeiten wie Öl und Wasser stabil ineinander zu verteilen bzw. die das Dispergieren eines pulverförmigen Feststoffes in einem flüssigen Medium verbessern. Bevorzugte erfindungsgemäße Hautbehandlungsmittel sind **dadurch gekennzeichnet, dass** die nichtionischen Öl-in-Wasser-Emulgatoren ausgewählt sind aus ethoxylierten C₈-C₂₄-Alkanolen mit durchschnittlich 10 - 100 Mol Ethylenoxid pro Mol, ethoxylierten C₈-C₂₄-Carbonsäuren mit durchschnittlich 10 - 100 Mol Ethylenoxid pro Mol, Silicon-Copolyolen mit Ethylenoxid-Einheiten oder mit Ethylenoxid- und Propylenoxid-Einheiten, Alkylmono- und -oligoglycosiden mit 8 bis 22 Kohlenstoffatomen im Alkylrest und deren ethoxylierten Analoga, ethoxylierten Sterinen, Partialestern von Polyglycerinen mit 2 bis 10 Glycerineinheiten und mit 1 bis 4 gesättigten oder ungesättigten, linearen oder verzweigten, gegebenenfalls hydroxylierten C₈ - C₃₀-Fettsäureresten verestert, sofern sie einen HLB-Wert von mehr als 7 aufweisen, sowie Mischungen der vorgenannten Substanzen. Die ethoxylierten C₈-C₂₄-Alkanole haben die Formel R¹O(CH₂CH₂O)ₙH, wobei R¹ steht für einen linearen oder verzweigten Alkyl- und/oder Alkenylrest mit 8 - 24 Kohlenstoffatomen und n, die mittlere Anzahl der Ethylenoxid-Einheiten pro Molekül, für Zahlen von 10 - 100, vorzugsweise 10 - 30 Mol Ethylenoxid an 1 Mol Caprylalkohol, 2-Ethylhexylalkohol, Caprinalkohol, Laurylalkohol, Isotridecylalkohol, Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen. Auch Addukte von 10 - 100 Mol Ethylenoxid an technische Fettalkohole mit 12 - 18 Kohlenstoffatomen, wie beispielsweise Kokos-, Palm-, Palmkern- oder Talgfettalkohol, sind geeignet. Die ethoxylierten C₈-C₂₄-Carbonsäuren haben die Formel R¹(OCH₂CH₂)ₙOH, wobei R¹ steht für einen linearen oder verzweigten gesättigten oder ungesättigten Acylrest mit 8 -24 Kohlenstoffatomen und n, die mittlere Anzahl der Ethylenoxid-Einheiten pro Molekül, für Zahlen von 10 - 100, vorzugsweise 10 - 30 Mol Ethylenoxid an 1 Mol Caprylsäure, 2-Ethylhexansäure, Caprinsäure, Laurinsäure, Isotridecansäure, Myristinsäure, Cetylsäure, Palmitoleinsäure, Stearinsäure, Isostearinsäure, Ölsäure, Elaidinsäure, Petroselinsäure, Arachinsäure, Gadoleinsäure, Behensäure, Erucasäure und Brassidinsäure sowie deren technische Mischungen. Auch Addukte von 10 - 100 Mol Ethylenoxid an technische Fettsäuren mit 12 - 18 Kohlenstoffatomen, wie Kokos-, Palm-, Palmkern- oder Talgfettsäure, sind geeignet. Besonders bevorzugt sind PEG-50-monostearat, PEG-100-monostearat, PEG-50-monooleat, PEG-100-monooleat, PEG-50-monolaurat und PEG-100-monolaurat. Besonders bevorzugt eingesetzt werden die C₁₂-C₁₈-Alkanole oder die C₁₂-C₁₈-Carbonsäuren mit jeweils 10 - 30 Einheiten Ethylenoxid pro Molekül sowie Mischungen dieser Substanzen, insbesondere Ceteth-12, Ceteth-20, Ceteth-30, Steareth-12, Steareth-20, Steareth-30, Laureth-12 und Beheneth-20. Weiterhin werden vorzugsweise C₈ - C₂₂-Alkylmono- und -oligoglycoside eingesetzt. C₈-C₂₂-Alkylmono- und -oligoglycoside stellen bekannte, handelsübliche Tenside und Emulgatoren dar. Ihre Herstellung erfolgt insbesondere durch Umsetzung von Glucose oder Oligosacchariden mit primären Alkoholen mit 8 - 22 Kohlenstoffatomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis etwa 8, vorzugsweise 1 - 2, geeignet sind. Der Oligomerisierungsgrad ist dabei ein statistischer Mittelwert, dem eine für solche technischen Produkte übliche Homologenverteilung zugrunde liegt. Produkte, die unter dem Warenzeichen Plantacare^{®} erhältlich sind, enthalten eine glucosidisch gebundene C₈-C₁₆-Alkylgruppe an einem Oligoglucosidrest, dessen mittlerer Oligomerisationsgrad bei 1 -2, insbesondere bei 1,1 - 1,4, liegt. Besonders bevorzugte C₈ - C₂₂-Alkylmono- und -oligoglycoside sind ausgewählt aus Octylglucosid, Decylglucosid, Laurylglucosid, Palmitylglucosid, Isostearylglucosid, Stearylglucosid, Arachidylglucosid und Behenylglucosid sowie Mischungen hiervon. Auch die vom Glucamin abgeleiteten Acylglucamide sind als nicht-ionische Öl-in-Wasser-Emulgatoren geeignet. Auch ethoxylierte Sterine, insbesondere ethoxylierte Sojasterine, stellen erfindungsgemäß geeignete Öl-in-Wasser-Emulgatoren dar. Der Ethoxylierungsgrad muss größer als 5, bevorzugt mindestens 10 sein, um einen HLB-Wert größer 7 aufzuweisen. Geeignete Handelsprodukte sind z. B. PEG-10 Soy Sterol, PEG-16 Soy Sterol und PEG-25 Soy Sterol. Weiterhin werden vorzugsweise Partialester von Polyglycerinen mit 2 bis 10 Glycerineinheiten und mit 1 bis 4 gesättigten oder ungesättigten, linearen oder verzweigten, gegebenenfalls hydroxylierten C₈ - C₃₀-Fettsäureresten verestert, eingesetzt, sofern sie einen HLB-Wert von mehr als 7 aufweisen. Besonders bevorzugt sind Diglycerinmonocaprylat, Diglycerinmonocaprat, Diglycerinmonolaurat, Triglycerinmonocaprylat, Triglycerinmonocaprat, Triglycerinmonolaurat, Tetraglycerinmonocaprylat, Tetraglycerinmonocaprat, Tetraglycerinmonolaurat, Pentaglycerinmonocaprylat, Pentaglycerinmonocaprat, Pentaglycerinmonolaurat, Hexaglycerinmonocaprylat, Hexaglycerinmonocaprat, Hexa glycerinmonolaurat, Hexaglycerinmonomyristat, Hexaglycerinmonostearat, Decaglycerinmonocaprylat, Decaglycerinmonocaprat, Decaglycerinmonolaurat, Decaglycerinmonomyristat, Decaglycerinmonoisostearat, Decaglycerinmonostearat, Decaglycerinmonooleat, Decaglycerinmonohydroxystearat, Decaglycerindicaprylat, Decaglycerindicaprat, Decaglycerindilaurat, Decaglycerindimyristat, Decaglycerindiisostearat, Decaglycerindistearat, Decaglycerindioleat, Decaglycerindihydroxystearat, Decaglycerintricaprylat, Decaglycerintricaprat, Decaglycerintrilaurat, Decaglycerintrimyristat, Decaglycerintriisostearat, Decaglycerintristearat, Decaglycerintrioleat und Decaglycerintrihydroxystearat. Besonders bevorzugte erfindungsgemäße Hautbehandlungsmittel sind dadurch gekennzeichnet, dass der nichtionische Öl-in-Wasser-Emulgator in einer Gesamtmenge von 0,5 - 10 Gew.-%, besonders bevorzugt 1 - 4 Gew.-% und außerordentlich bevorzugt 1,5 - 3 Gew.-%, bezogen auf die Gesamtzusammensetzung, enthalten ist. Besonders vorteilhaft einsetzbare zusätzliche Wasser-in-Öl-Emulgatoren sind Stearylalkohol, Cetylalkohol, Glycerylmonostearat, insbesondere in Form der Handelsprodukte Cutina^{®} GMS und Cutina^{®} MD (ex Cognis), Glyceryldistearat, Glycerylmonocaprinat, Glycerylmonocaprylat, Glycerylmonolaurat, Glycerylmonomyristat, Glycerylmonopalmitat, Glycerylmonohydroxystearat, Glycerylmonooleat, Glycerylmonolanolat, Glyceryldimyristat, Glyceryldipalmitat, Glyceryldioleat, Propylenglycolmonostearat, Propylenglycolmonolaurat, Sorbitanmonocaprylat, Sorbitanmonolaurat, Sorbitanmonomyristat, Sorbitanmonopalmitat, Sorbitanmonostearat, Sorbitansesquistearat, Sorbitandistearat, Sorbitandioleat, Sorbitansesquioleat, Saccharosedistearat, Arachidylalkohol, Behenylalkohol, Polyethylenglycol(2)stearylether (Steareth-2), Steareth-5, Oleth-2, Diglycerinmonostearat, Diglycerinmonoisostearat, Diglycerinmonooleat, Diglycerindihydroxystearat, Diglycerindistearat, Diglycerindioleat, Triglycerindistearat, Tetraglycerinmonostearat, Tetraglycerindistearat, Tetraglycerintristearat, Decaglycerinpentastearat, Decaglycerinpentahydroxystearat, Decaglycerinpentaisostearat, Decaglycerinpentaoleat, Soy Sterol, PEG-1 Soy Sterol, PEG-5 Soy Sterol, PEG-2-monolaurat und PEG-2-monostearat. Besonders bevorzugte erfindungsgemäße Hautbehandlungsmittel sind **dadurch gekennzeichnet, dass** mindestens ein Wasser-in-Öl-Emulgator in einer Gesamtmenge von 0,1 - 15 Gew.-%, bevorzugt 0,5 - 8,0 Gew.-%, und besonders bevorzugt 1 - 4 Gew.-%, bezogen auf die Gesamtzusammensetzung, enthalten sind. Weiterhin können auch Mengen von 2 - 3 Gew.-%, bezogen auf das Gesamtgewicht der Zusammensetzung, erfindungsgemäß außerordentlich bevorzugt sein.
f) Enthaarungsmittel und Wirkstoffe mit Haarwuchs inhibierender Wirkung. Diese dienen der vorzugsweise selektiven Entfernung von Körperbehaarung bzw. der Verhinderung des Nachwachsens von entferntem, beispielsweise rasiertem oder epiliertem, Haar.
g) Feuchtigkeitsspender
   Diese können einen Beitrag zur Erhaltung oder Wiederherstellung der Hautfeuchtigkeit leisten und dem Austrocknen der Haut entgegenwirken. Neben den oben erwähnten Emollients eignen sich hierzu vor wasserlösliche mehrwertige C₂ - C₉-Alkanole mit 2 - 6 Hydroxylgruppen und/oder wasserlösliche Polyethylenglycole mit 3 - 20 Ethylenoxid-Einheiten sowie Mischungen hiervon. Bevorzugt sind diese Komponenten ausgewählt aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit sowie Mischungen der vorgenannten Substanzen. Geeignete wasserlösliche Polyethylenglycole sind ausgewählt aus PEG-3, PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 bevorzugt sind. Auch Zucker und bestimmte Zuckerderivate wie Fucose, Rhamnose, Fructose, Glucose, Maltose, Maltitol, Mannit, Inosit, Sucrose, Trehalose und Xylose sind erfindungsgemäß geeignet. Bevorzugte erfindungsgemäße Hautbehandlungsmittel sind dadurch gekennzeichnet, dass das mindestens eine wasserlösliche mehrwertige C₂ - C₉-Alkanol mit 2 - 6 Hydroxylgruppen und/oder mindestens eine wasserlösliche Polyethylenglycol mit 3 - 20 Ethylenoxid-Einheiten ausgewählt ist aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit sowie Mischungen der vorgenannten Substanzen. Besonders bevorzugte erfindungsgemäße Hautbehandlungsmittel sind dadurch gekennzeichnet, dass das mindestens eine wasserlösliche mehrwertige C₂ - C₉-Alkanol mit 2 - 6 Hydroxylgruppen und/oder mindestens eine wasserlösliche Polyethylenglycol mit 3 - 20 Ethylenoxid-Einheiten insgesamt in Mengen von 3 - 30 Gew.-%, bevorzugt 8 - 25 Gew.-%, besonders bevorzugt 10 - 18 Gew.-%, jeweils bezogen auf die Gesamtzusammensetzung, enthalten ist.
h) Filmbildner
   Diese sind dazu im Stande, einen schützenden, stabilisierenden Film auf Oberflächen, vorzugsweise Haut, Haar oder Nägeln, zu erzeugen. Mit Bezug auf die erfindungsgemäßen Mittel wirkt dieser Film vorzugsweise feuchtigkeitsspeichernd. Bevorzugte feuchtigkeitsspeichernde Filmbildner sind ausgewählt aus Glycosaminoglycanen, besonders bevorzugt Hyaluronsäure, Dextran, Dextransulfat, Chondroitin-4-sulfat und Chondroitin-6-sulfat, und Polysacchariden, die mindestens einen Desoxyzucker-Baustein enthalten Erfindungsgemäß bevorzugte Desoxyzucker sind ausgewählt aus Rhamnose und Fucose. Ein erfindungsgemäß besonders bevorzugtes, Desoxyzucker-Bausteine enthaltendes Polysaccharid ist das Handelsprodukt Fucogel^{®} von Solabia mit der INCI-Bezeichnung Biosaccharide Gum-1. Ein weiteres erfindungsgemäß besonders bevorzugtes, Desoxyzucker-Bausteine enthaltendes Polysaccharid ist das Handelsprodukt Rhamnosoft^{®} von Solabia mit der INCI-Bezeichnung Biosaccharide Gum-2. Ein erfindungsgemäß besonders bevorzugtes, Desoxyzucker-Bausteine enthaltendes Polysaccharid ist das Handelsprodukt Fucogenol^{®} von Solabia mit der INCI-Bezeichnung Biosaccharide Gum-3. Ein erfindungsgemäß besonders bevorzugtes, Desoxyzucker-Bausteine enthaltendes Polysaccharid ist das Handelsprodukt Glycofilm^{®} von Solabia mit der INCI-Bezeichnung Biosaccharide Gum-4. Erfindungsgemäß bevorzugt sind weiterhin Mischungen der vorgenannten, mindestens einen Desoxyzucker-Baustein enthaltenden Polysaccharide, beispielsweise die Mischung aus Biosaccharide Gum-2 und Biosaccharide Gum-3, erhältlich als Handelsprodukt Elastinol plus^{®} von Solabia. Erfindungsgemäß besonders bevorzugte kosmetische oder dermatologische Zusammensetzungen sind dadurch gekennzeichnet, dass sie mindestens einen Desoxyzucker und/ oder mindestens ein Desoxyzucker-Bausteine enthaltendes Polysaccharid in Gesamtmengen von 0,001 - 5 Gew.-%, bevorzugt 0,01 - 2 Gew.-% und besonders bevorzugt 0,1 - 1 Gew.-%, jeweils bezogen auf die gesamte erfindungsgemäße Zusammensetzung, enthalten.
i) Konservierungsstoffe
   Diese werden kosmetischen Mitteln zugesetzt, um sie vor der schadhaften Einwirkung von Mikroorganismen (Bakterien, Pilze, Hefen) zu bewahren und damit ihren Verderb zu vermeiden. Zahlreiche Konservierungsstoffe haben zwangsläufig auch desodorierende Eigenschaften, da sie auch gegen geruchsverursachende Bakterien antimikrobiell wirken, so dass einige Stoffe beiden Gruppen angehören. Für Kosmetika als Konservierungsstoffe bevorzugt geeignet sind beispielsweise Benzoesäure und deren Derivate (z. B. Propyl-, Phenyl- und Butylbenzoat, Ammonium-, Natrium, Kalium- und Magnesiumbenzoat), Propionsäure und deren Derivate (z. B. Ammonium-, Natrium-, Kalium-, und Magnesiumpropionat), Salicylsäure und deren Derivate (z. B. Natrium-, Kalium- und Magnesiumsalicylat), 4-Hydroxybenzoesäure und deren Ester und Alkalimetallsalze (z. B. Methyl-, Ethyl-, Propyl-, Isopropyl-, Butyl-, Isobutyl-, Isodecyl-, Phenyl-, Phenoxyethyl- und Benzylparaben, Hexamidinparaben und -diparaben, Natrium- und Kaliumparaben, Natrium- und Kaliummethylparaben, Kaliumbutylparaben, Natrium- und Kaliumpropylparaben), Alkohole und deren Salze (z.B. Ethanol, Propanol, Isopropanol, Benzylalkohol, Phenethylalkohol, Phenol, Kaliumphenolat, Phenoxyethanol, Phenoxyisopropanol, o-Phenylphenol), und weitere. Die Menge der Konservierungsmittel in den bevorzugten erfindungsgemäßen Zusammensetzungen beträgt 0,001 - 10 Gew.-%, vorzugsweise 0,01 -5 Gew.-% und insbesondere 0,1 - 3 Gew.-%, bezogen auf das Gesamtgewicht der Zusammensetzung.
j) Hautkühlende Wirkstoffe
   Erfindungsgemäß besonders bevorzugte Zusammensetzungen enthalten weiterhin bevorzugt mindestens einen hautkühlenden Wirkstoff. Erfindungsgemäß geeignete hautkühlende Wirkstoffe sind beispielsweise Menthol, Isopulegol sowie Mentholderivate, z. B. Menthyllactat, Menthylglycolat, Menthylpyrrolidoncarbonsäure und andere.
k) pH-Wert-Regler/Puffersubstanzen
   Diese können z.B. in Kosmetika dazu dienen, um einen gewünschten pH-Wert einzustellen bzw. zu stabilisieren.
l) Tenside/Waschaktive Substanzen
   Dies sind grenzflächenaktive Verbindungen, die Reinigungszwecken oder aber der besseren Abwaschbarkeit der erfindungsgemäßen Mittel dienen.
m) Treibgase
   Dies sind gasförmige Substanzen, mit denen die erfindungsgemäßen kosmetischen Mittel unter Druck in druckbeständigen Behältern gesetzt werden können, um den Inhalt dann bei Druckentlastung hervorzubringen. Erfindungsgemäß besonders bevorzugte Zusammensetzungen, die als treibgasgetriebenes Aerosol konfektioniert sind, enthalten mindestens ein Treibmittel. Bevorzugte Treibmittel (Treibgase) sind Propan, Propen, n-Butan, iso-Butan, isoButen, n-Pentan, Penten, iso-Pentan, iso-Penten, Methan, Ethan, Dimethylether, Stickstoff, Luft, Sauerstoff, Lachgas, 1,1,1,3-Tetrafluorethan, Heptafluoro-n-propan, Perfluorethan, Monochlordifluormethan, 1,1-Difluorethan, und zwar sowohl einzeln als auch in Kombination. Auch hydrophile Treibgase, wie z. B. Kohlendioxid, können vorteilhaft im Sinne der vorliegenden Erfindung eingesetzt werden, wenn der Anteil an hydrophilen Gasen gering gewählt wird und lipophiles Treibgas (z. B. Propan/Butan) im Überschuss vorliegt. Besonders bevorzugt sind Propan, n-Butan, iso-Butan sowie Mischungen dieser Treibgase. Es hat sich gezeigt, dass der Einsatz von n-Butan als einzigem Treibgas erfindungsgemäß besonders bevorzugt sein kann. Die Menge der Treibmittel beträgt bevorzugt 20 - 80 Gew.%, besonders bevorzugt 30 - 70 Gew.% und außerordentlich bevorzugt 40 - 50 Gew.-%, jeweils bezogen auf das Gesamtgewicht der Zubereitung, bestehend aus der erfindungsgemäßen Zusammensetzung und dem Treibmittel
n) Trübungsmittel
   Diese kann man vorzugsweise durchsichtigen oder durchscheinenden Produkten beigeben, um sie undurchdringlicher für sichtbares Licht oder lichtnahe Strahlung zu machen.

Vorgenannte Inhaltsstoffe können gemäß weiterer bevorzugter Ausführungsformen als einziger Zusatz oder in beliebigen Kombinationen in den erfindungsgemäßen Mitteln enthalten sein. Nachfolgend werden einige besonders bevorzugte Stoffe ausführlicher beschrieben - dabei wird überwiegend auf erfindungsgemäße Hautbehandlungsmittel Bezug genommen. Selbstverständlich können die genannten Stoffe auch in Haarbehandlungsmitteln, anderen kosmetischen mitteln oder den vorstehend genannten nicht-kosmetischen Mitteln wie Weichspülern oder WC-Reinigern enthalten sein. Insofern ist der nachfolgend verwendete Begriff "Hautbehandlungsmittel" nicht beschränkend zu verstehen.

Im folgenden werden bevorzugte Inhaltsstoffe der erfindungsgemäßen Mittel näher beschrieben.

Anionische Tenside sind bevorzugt in den erfindungsgemäßen Mitteln enthalten. Als anionische Tenside werden beispielsweise solche vom Typ der Sulfonate und Sulfate eingesetzt. Als Tenside vom Sulfonat-Typ kommen dabei vorzugsweise C₉₋₁₃-Alkylbenzolsulfonate, Olefinsulfonate, d.h. Gemische aus Alken- und Hydroxyalkansulfonaten sowie Disulfonaten, wie man sie z.B. aus C₁₂₋₁₈-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch Alkansulfonate, die aus C₁₂₋₁₈-Alkanen beispielsweise durch Sulfochlorierung oder Sulfoxidation mit anschließender Hydrolyse bzw. Neutralisation gewonnen werden. Ebenso sind auch die Ester von α-Sulfofettsäuren (Estersulfonate), z.B. die α-sulfonierten Methylester der hydrierten Kokos-, Palmkern- oder Talgfettsäuren geeignet. Weitere geeignete Aniontenside sind sulfierte Fettsäureglycerinester. Unter Fettsäureglycerinestern sind die Mono-, Di- und Triester sowie deren Gemische zu verstehen, wie sie bei der Herstellung durch Veresterung von einem Monoglycerin mit 1 bis 3 Mol Fettsäure oder bei der Umesterung von Triglyceriden mit 0,3 bis 2 Mol Glycerin erhalten werden. Bevorzugte sulfierte Fettsäureglycerinester sind dabei die Sulfierprodukte von gesättigten Fettsäuren mit 6 bis 22 Kohlenstoffatomen, beispielsweise der Capronsäure, Caprylsäure, Caprinsäure, Myristinsäure, Laurinsäure, Palmitinsäure, Stearinsäure oder Behensäure.

Als Alk(en)ylsulfate werden die Alkali- und insbesondere die Natriumsalze der Schwefelsäurehalbester der C₁₂-C₁₈-Fettalkohole, beispielsweise aus Kokosfettalkohol, Talgfettalkohol, Lauryl-, Myristyl-, Cetyl- oder Stearylalkohol oder der C₁₀-C₂₀-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. Aus waschtechnischem Interesse sind die C₁₂-C₁₆-Alkylsulfate und C₁₂-C₁₅-Alkylsulfate sowie C₁₄-C₁₅-Alkylsulfate bevorzugt. Auch 2,3-Alkylsulfate, als Handelsprodukte der Shell Oil Company unter dem Namen DAN^{®} erhalten werden können, sind geeignete Aniontenside. Auch die Schwefelsäuremonoester der mit 1 bis 6 Mol Ethylenoxid ethoxylierten geradkettigen oder verzweigten C₇₋₂₁-Alkohole, wie 2-Methyl-verzweigte C₉₋₁₁-Alkohole mit im Durchschnitt 3,5 Mol Ethylenoxid (EO) oder C₁₂₋₁₈-Fettalkohole mit 1 bis 4 EO, sind geeignet. Sie werden insbesondere in Reinigungsmitteln aufgrund ihres hohen Schaumverhaltens vorzugsweise nur in relativ geringen Mengen, z.B. in Mengen von 1 - 5 Gew.-%, eingesetzt. Vorzugsweise können die erfindungsgemäßen Mittel frei von Schwefelsäuremonoester sein.

Eine weitere Klasse von Aniontensiden ist die durch Umsetzung von Fettalkoholethoxylaten mit Natriumchloracetat in Gegenwart basischer Katalysatoren zugängliche Klasse der Ethercarbonsäuren. Sie haben die allgemeine Formel: R¹⁰ O-(CH₂-CH₂-O)ₚ-CH₂-COOH mit R¹⁰ = C₁-C₁₈ und *p* = 0,1 bis 20. Ethercarbonsäuren sind wasserhärteunempfindlich und weisen ausgezeichnete Tensideigenschaften auf. Geeignete anionische Tenside sind beispielsweise auch die Partialester von Di- oder Polyhydroxyalkanen, Mono- und Disacchariden, Polyethylenglycolen mit den EO-Addukten von Maleinsäureanhydrid an mindestens einfach ungesättigte Carbonsäuren mit einer Kettenlänge von 10 bis 25 Kohlenstoffatomen mit einer Säurezahl von 10 bis 140.

Bei den Salzen handelt es sich bevorzugt um Alkalimetallsalze, Ammoniumsalze sowie Mono-, Di- bzw. Trialkanolammoniumsalze, beispielsweise Mono-, Di- bzw. Triethanolammoniumsalze, insbesondere um Lithium-, Natrium-, Kalium- oder Ammoniumsalze, besonders bevorzugt Natrium- oder Ammoniumsalze, äußerst bevorzugt Natriumsalze.

Eine weitere geeignete Verbindungsklasse sind Aniontensid-Kation-Komplexe, wobei das Kation selbst ursprünglich tensidische Eigenschaften aufweist. Beispiele sind Umesterungsprodukte von LAS-Säure mit Aminen, Aminderivaten, enthaltend eine C-Kette mit > 2 C-Atomen, vorzugsweise C₁₂-C₁₆. Diese können z.B. am Stickstoff oxidiert sein, d.h. z.B. Umesterung von LAS-Säure mit Aminoxid C₁₂₋₁₄.

Der Gehalt des erfindungsgemäßen Mittels an anionischen Tensiden, vorzugsweise an den genannten anionischen Tensiden, kann in weiten Bereichen variieren, je nachdem welchem Zweck das betreffende Mittel dient. So kann ein erfindungsgemäßes Mittel sehr große Mengen Aniontensid enthalten, vorzugsweise bis zu einer Größenordnung von bis zu 40, 50 oder 60 Gew.- oder mehr. Ebenso kann ein erfindungsgemäßes Mittel nur sehr geringe Mengen Aniontensid enthalten, beispielsweise weniger als 15 oder 10 Gew.-% oder weniger als 5 Gew.-% oder noch weniger. Vorteilhafterweise sind in den erfindungsgemäßen Mitteln jedoch Aniontenside in Mengen von 0,1 bis 40 Gew.-% und insbesondere 5 bis 30 Gew.-% enthalten, wobei Konzentrationen oberhalb von 10 Gew.-% und sogar oberhalb von 15 Gew.-% besondere Bevorzugung finden. Nach einer bevorzugten Ausführungsform enthält das erfindungsgemäße Mittel anionische Tenside, vorzugsweise in Mengen von zumindest 0,01 Gew.-%, bezogen auf das gesamte Mittel. Nach einer anderen bevorzugten Ausführungsform kann das erfindungsgemäße Mittel frei von Aniontensid sein.

Zusätzlich zu den genannten anionischen Tensiden, aber auch unabhängig von diesen, können in den erfindungsgemäßen Mitteln Seifen enthalten sein. Geeignet sind insbesondere gesättigte Fettsäureseifen, wie die Salze der Laurinsäure, Myristinsäure, Palmitinsäure, Stearinsäure, hydrierte Erucasäure und Behensäure sowie insbesondere aus natürlichen Fettsäuren, z.B. Kokos-, Palmkern- oder Talgfettsäuren, abgeleitete Seifengemische. Der Gehalt des Mittels an Seifen beträgt, unabhängig von anderen Aniontensiden, vorzugsweise nicht mehr als 3 Gew.-% und insbesondere 0,5 bis 2,5 Gew.-%, bezogen auf das gesamte Mittel. Nach einer anderen bevorzugten Ausführungsform ist das erfindungsgemäße Mittel frei von Seife.

Die anionischen Tenside und Seifen können in Form ihrer Natrium-, Kalium- oder Ammoniumsalze sowie als lösliche Salze organischer Basen, wie Mono-, Di- oder Triethanolamin, vorliegen. Vorzugsweise liegen sie in Form ihrer Natrium- oder Kaliumsalze, insbesondere in Form der Natriumsalze vor. Anionische Tenside und Seifen können auch in situ hergestellt werden, indem in die sprühzutrocknende Zusammensetzung die Aniontensidsäuren und gegebenenfalls Fettsäuren eingebracht werden, welche dann durch die Alkaliträger in der sprühzutrocknenden Zusammensetzung neutralisiert werden.

Vorteilhafterweise können nichtionische Tenside in den erfindungsgemäßen Mitteln ebenfalls enthalten sein, sowohl in festen wie in flüssigen Mitteln. Beispielsweise kann ihr Gehalt bis zu 2 oder 3 oder 5 Gew.-% betragen. Es können auch größere Mengen an nichtionischem Tensid enthalten sein, beispielsweise bis zu 5 Gew.-% oder 10 Gew.-% oder 15 Gew.-% oder 20 Gew.-%, 30 Gew.-%, 40 Gew.-%, 50 Gew.-% oder sogar darüber hinaus, falls es zweckmäßig ist. Sinnvolle Untergrenzen können bei Werten von 0,01, 0,1, 1, 2, 3 oder 4 Gew.-% liegen.

Vorzugsweise sind die nichtionischen Tenside aber in größeren Mengen, also bis zu 50 Gew.-%, vorteilhafterweise von 0,1 bis 40 Gew.-%, besonders bevorzugt von 0,5 bis 30 und insbesondere von 2 bis 25 Gew.-%, jeweils bezogen auf das gesamte Mittel, enthalten. Nach einer bevorzugten Ausführungsform enthält das erfindungsgemäße Mittel nichtionische Tenside, vorzugsweise in Mengen von zumindest 0,1 Gew.-%, bezogen auf das gesamte Mittel. Nach einer anderen bevorzugten Ausführungsform kann das erfindungsgemäße Mittel frei von Niotensid sein. Vorteilhafterweise können alle aus dem Stand der Technik bekannten nichtionischen Tenside in den erfindungsgemäßen Mitteln enthalten sein. Bevorzugte Niotenside werden weiter unten vorgestellt.

Die erfindungsgemäßen Mittel können vorzugsweise auch mindestens ein kationisches Tensid enthalten. Geeignete Kationtenside sind beispielsweise oberflächenaktive quaternäre Verbindungen, insbesondere mit einer Ammonium-, Sulfonium-, Phosphonium-, Jodonium- oder Arsoniumgruppe. Durch den Einsatz von quaternären oberflächenaktiven Verbindungen mit antimikrobieller Wirkung kann das Mittel mit einer antimikrobiellen Wirkung ausgestaltet werden bzw. dessen gegebenenfalls aufgrund anderer Inhaltsstoffe bereits vorhandene antimikrobielle Wirkung verbessert werden.

Besonders bevorzugte kationische Tenside sind die quaternären, z.T. antimikrobiell wirkenden Ammoniumverbindungen (QAV; *INCI* Quaternary Ammonium Compounds) gemäß der allgemeinen Formel (R^{I})(R^{II})(R^{III})(R^{IV})N⁺ X⁻, in der R^{I} bis R^{IV} gleiche oder verschiedene C₁₋₂₂-Alkylreste C₇₋₂₈-Aralkyl-reste oder heterozyklische Reste, wobei zwei oder im Falle einer aromatischen Einbindung wie im Pyridin sogar drei Reste gemeinsam mit dem Stickstoffatom den Heterozyklus, z.B. eine Pyridinium- oder Imidazoliniumverbindung, bilden, darstellen und X⁻ Halogenidionen, Sulfationen, Hydroxidionen oder ähnliche Anionen sind. Für eine optimale antimikrobielle Wirkung weist vorzugsweise wenigstens einer der Reste eine Kettenlänge von 8 bis 18, insbesondere12 bis 16, C-Atomen auf.

Zur Vermeidung möglicher Inkompatibilitäten der antimikrobiellen kationischen Tenside mit in dem erfindungsgemäßen Mittel enthaltenen anionischen Tensiden werden möglichst aniontensidverträgliches und/oder ggf. möglichst wenig kationisches Tensid eingesetzt oder in einer besonderen Ausführungsform der Erfindung gänzlich auf kationische Tenside verzichtet.

Weiter unten werden insbesondere im Zusammenhang mit Konditioniermitteln und Weichmachern weitere kationische Tenside, so auch quartäre Ammoniumverbindungen beschrieben. Auch diese können vorzugsweise in den erfindungsgemäßen Mitteln enthalten sein.

Die erfindungsgemäßen Mittel können ein oder mehrere kationische Tenside enthalten, bevorzugt in Mengen, bezogen auf die Gesamtzusammensetzung, von 0 bis 30 Gew.-%, noch bevorzugter größer 0 bis 20 Gew.-%, vorzugsweise 0,01 bis 10 Gew.-%, insbesondere 0,1 bis 5 Gew.-%. Geeignete Mindestwerte können auch bei 0,5, 1, 2 oder 3 Gew.-% liegen. Nach einer bevorzugten Ausführungsform enthält das erfindungsgemäße Mittel kationische Tenside, vorzugsweise in Mengen von zumindest 0,1 Gew.-%, bezogen auf das gesamte Mittel. Nach einer anderen bevorzugten Ausführungsform kann das erfindungsgemäße Mittel frei von Kationtensid sein. Ebenso können die erfindungsgemäßen Mittel auch mindestens ein amphoteres Tensid enthalten. Diese werden weiter unten insbesondere im Zusammenhang mit Konditioniermitteln und Weichmachern noch näher beschrieben.

Die erfindungsgemäßen Mittel können ein oder mehrere amphotere Tenside enthalten, vorteilhafterweise in Mengen, bezogen auf die Gesamtzusammensetzung, von 0 bis 30 Gew.-%, noch vorteilhafter größer 0 bis 20 Gew.-%, vorzugsweise 0,01 bis 10 Gew.-%, insbesondere 0,1 bis 5 Gew.-%. Nach einer anderen bevorzugten Ausführungsform kann das erfindungsgemäße Mittel frei von amphoteren Tensiden sein.

Nach einer bestimmten Ausführungsform können die erfindungsgemäßen Mittel nur sehr wenig Gesamttensid enthalten, z.B. kann die Gesamttensidmenge unter 20 Gew.-%, 15 Gew.-%, 10 Gew.-% oder 5 Gew.-%, vorteilhafterweise sogar unter 3 Gew.-% oder unter 1 Gew.-%, insbesondere sogar unter 0,5 Gew.-% oder unter 0,1 Gew.-% liegen, Gew.-% jeweils bezogen auf das gesamte Mittel. Vorzugsweise beträgt der Gesamttensidgehalt aber zumindest 0,01 Gew.-%, 0,1 Gew-% oder 1 Gew.-%, bezogen auf das gesamte Mittel.

Lösliche Komplexbildner kann das erfindungsgemäße Mittel vorzugsweise in Mengen von 0,1 - 30 Gew.-%, bevorzugt 5 - 25 Gew.-% und besonders bevorzugt 10 - 20 Gew.-%, bezogen auf das Gesamtgewicht des Mittel, enthalten, wobei Ethylendiamintetraeesigsäure und ihre Natriumsalze als lösliche Komplexbildner besonders bevorzugt sind. Es kann aber vorteilhafterweise auch vorgesehen sein, dass das erfindungsgemäße Mittel weniger als 10 Gew.-%, beispielsweise weniger als 5 Gew.-% lösliche Komplexbildner enthält. Geeignet sind beispielsweise Citrate, SKS-6, Citronensäure, MGDA (methyl glycine di-acetic acid), Triphosphate, Phosphonate, aliphatische Dicarbonsäuren (z.B. Adipin-, Glutar-, Bernsteinsäure).

Nach einer anderen bevorzugten Ausführungsform kann das erfindungsgemäße Mittel frei von löslichen Komplexbildnern sein.

Weitere geeignete organische Komplexbildner sind Dextrine, beispielsweise Oligomere bzw. Polymere von Kohlenhydraten, die durch partielle Hydrolyse von Stärken erhalten werden können. Auch Oxydisuccinate und andere Derivate von Disuccinaten, vorzugsweise Ethylendiamindisuccinat, sind weitere geeignete Cobuilder. Dabei wird Ethylendiamin-N,N'-di-succinat (EDDS) bevorzugt in Form seiner Natrium- oder Magnesiumsalze verwendet. Weiterhin bevorzugt sind in diesem Zusammenhang auch Glycerindisuccinate und Glycerintrisuccinate. Geeignete Einsatzmengen liegen beispielsweise bei 3 bis 15 Gew.-%, bezogen auf das gesamte Mittel. Weitere brauchbare organische Co-Komplexbildner sind beispielsweise acetylierte Hydroxycarbonsäuren bzw. deren Salze, welche gegebenenfalls auch in Lactonform vorliegen können und welche mindestens 4 Kohlenstoffatome und mindestens eine Hydroxygruppe sowie maximal zwei Säuregruppen enthalten. Eine weitere Substanzklasse mit Co-Komplexbildner-Eigenschaften stellen die Phosphonate dar. Dabei handelt es sich insbesondere um Hydroxyalkan- bzw. Aminoalkanphosphonate. Unter den Hydroxyalkanphosphonaten ist das 1-Hydroxyethan-1,1-diphosphonat (HEDP) von besonderer Bedeutung als Co-Komplexbildner. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, z.B. als Hexanatriumsalz der EDTMP bzw. als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Mittel auch Bleiche enthalten, bevorzugt sein, Aminoalkanphosphonate, insbesondere DTPMP, einzusetzen, oder Mischungen aus den genannten Phosphonaten zu verwenden. Bevorzugte organische Komplexbildner sind beispielsweise die in Form ihrer Alkali- und insbesondere Natriumsalze einsetzbaren Polycarbonsäuren, wie Citronensäure, Adipinsäure, Bernsteinsäure, Glutarsäure, Weinsäure, Zuckersäuren, Aminocarbonsäuren, Nitrilotriessigsäure (NTA), sofern ein derartiger Einsatz aus ökologischen Gründen nicht zu beanstanden ist, sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen. Auch die Säuren an sich können eingesetzt werden. Die Säuren besitzen neben ihrer komplexierenden Wirkung typischerweise auch die Eigenschaft einer Säuerungskomponente und dienen somit auch zur Einstellung eines niedrigeren und milderen pH-Werts bevorzugter erfindungsgemäßer Hautbehandlungsmittel. Insbesondere sind hierbei Citronensäure, Bernsteinsäure, Glutarsäure, Adipinsäure, Gluconsäure und beliebige Mischungen aus diesen zu nennen.

Das erfindungsgemäße Mittel enthält bevorzugt mindestens einen Komplexbildner in einer Gesamtmenge von üblicherweise 0 - 20 Gew.-%, vorzugsweise 0,1 - 15 Gew.-%, insbesondere 0,5 - 10 Gew.-%, besonders bevorzugt 1 - 8 Gew.-%, äußerst bevorzugt 1,5 - 6 Gew.-%, bezogen auf das gesamte Mittel.

Es sei an dieser Stelle darauf hingewiesen, dass sich die Angabe Gew.-%, sofern es nicht anders angegeben ist, jeweils auf das gesamte Mittel bezieht.

Der Gehalt an Wasser in den erfindungsgemäßen Mitteln beträgt 20 - 95 Gew.-%, bevorzugt 30 - 90 Gew.-%, besonders bevorzugt 40 - 85 Gew.-%, außerordentlich bevorzugt 50 - 80 Gew.-%. Wassergehalte von 55 - 75 Gew.-%, 60 - 70 und 65 Gew.-% können ebenfalls bevorzugt sein. Nicht mit eingerechnet wurde hierbei das in einzelnen Rohstoffen, wie insbesondere den schweißhemmenden Aluminium- und/oder Zirconium-Verbindungen, vorhandene Kristallwasser.

In einer weiteren bevorzugten Ausführungsform enthält das erfindungsgemäße Hautbehandlungsmittel mindestens eine UV-absorbierende Substanz enthalten. Bei einem Einsatz in geringen Mengen (bis etwa 1 Gew.-%) dient die UV-absorbierende Substanz dazu, vorteilhafterweise die Lichtbeständigkeit sonstiger Rezepturbestandteile zu verbessern (Produktschutz). Bei einem Einsatz in höheren Mengen (ab mehr als 1 Gew.-%) dient die UV-absorbierende Substanz dazu, die behandelte Haut vor den schädlichen Auswirkungen der UV-Strahlung zu schützen. Unter UV-absorbierende Substanz sind organische und anorganische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. Verbindungen, die diese gewünschten Eigenschaften aufweisen, sind beispielsweise die durch strahlungslose Desaktivierung wirksamen Verbindungen und Derivate des Benzophenons mit Substituenten in 2- und/oder 4-Stellung. Weiterhin sind auch substituierte Benzotriazole, in 3-Stellung Phenylsubstituierte Acrylate (Zimtsäurederivate), gegebenenfalls mit Cyanogruppen in 2-Stellung, Salicylate sowie Naturstoffe wie Umbelliferon und die körpereigene Urocansäure geeignet. Besondere Bedeutung haben Biphenyl- und vor allem Stilbenderivate, kommerziell als Tinosorb^{®} FD oder Tinosorb^{®} FR ex Ciba erhältlich. Als UV-B-Absorber sind zu nennen 3-Benzylidencampher bzw. 3-Benzylidennorcampher und dessen Derivate, z.B. 3-(4-Methylbenzyliden)campher; 4-Aminobenzoesäurederivate, vorzugsweise 4-(Di-methylamino)benzoesäure-2-ethylhexylester, 4-(Dimethylamino)benzoe-säure-2-octylester und 4-(Dimethylamino)benzoesäureamylester; Ester der Zimtsäure, vorzugsweise 4-Meth-oxyzimtsäure-2-ethylhexylester, 4-Methoxyzimtsäurepropylester, 4-Methoxyzimtsäureiso-amyl-ester, 2-Cyano-3,3-phenylzimtsäure-2-ethylhexylester (Octocrylene); Ester der Salicylsäure, vorzugsweise Salicylsäure-2-ethylhexylester, Salicylsäure-4-isopropylbenzyl-ester, Salicylsäurehomomenthylester; Derivate des Benzophenons, vorzugsweise 2-Hy-droxy-4-methoxybenzophenon, 2-Hydroxy-4-methoxy-4`-methylbenzophenon, 2,2'-Dihydroxy-4-methoxybenzophenon; Ester der Benzalmalonsäure, vorzugsweise 4-Methoxy-benzmalonsäuredi-2-ethylhexylester; Triazinderivate, wie z.B. 2,4,6-Trianilino-(p-carbo-2'-ethyl-1'-hexyloxy)-1,3,5-triazin und Octyl Triazon, oder Dioctyl Butamido Triazone (Uvasorb® HEB); Propan-1,3-dione, wie z.B. 1-(4-tert.Butylphenyl)-3-(4'methoxyphenyl)propan-1,3-dion; Ketotricyclo(5.2.1.0)-decan-Derivate. Weiterhin geeignet sind 2-Phenylbenzimidazol-5-sul-fonsäure und deren Alkali-, Erdalkali-, Ammonium-, Alkylammonium-, Alkanolammonium- und Glucammoniumsalze; Sulfonsäurederivate von Benzophenonen, vorzugsweise 2-Hy-droxy-4-methoxybenzophenon-5-sulfon-säure und ihre Salze; Sulfonsäurederivate des 3-Benzylidencamphers, wie z.B. 4-(2-Oxo-3-bor-nylidenmethyl)benzol-sulfonsäure und 2-Methyl-5-(2-oxo-3-bornyliden)sulfonsäure und deren Salze.

Als typische UV-A-Filter kommen insbesondere Derivate des Benzoylmethans in Frage, wie z.B. 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen. Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse, vorzugsweise nanoisierte Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zink-stearat eingesetzt werden. Die Oxide und Salze werden in Form der Pigmente bereits für hautpflegende und hautschützende Emulsionen und dekorative Kosmetik verwendet. Die Partikel sollten dabei einen mittleren Durchmesser von weniger als 100 nm, vorzugsweise von 5 bis 50 nm und insbesondere von 15 bis 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. Ebenfalls bevorzugt kann mikronisiertes Zinkoxid verwendet werden. Weitere geeignete UV-Lichtschutzfilter sind dem einschlägigen Stand der Technik zu entnehmen.

Die mindestens eine UV-absorbierende Substanz ist in bevorzugten erfindungsgemäßen Hautbehandlungsmitteln in einer Gesamtmenge von 0,01 Gew.-% bis 20 Gew.-%, besonders bevorzugt 1 - 10 Gew.-%, außerordentlich bevorzugt 2 - 7 Gew.-%, enthalten.

In einer weiteren bevorzugten Ausführungsform enthalten die erfindungsgemäßen Mittel neben Wasser mindestens ein flüssiges hydrophiles organisches Lösemittel. Bevorzugte Lösemittel stammen z.B. aus der Gruppe ein- oder mehrwertigen Alkohole, Alkanolamine oder Glycolether, sofern sie im angegebenen Konzentrationsbereich mit Wasser mischbar sind. Vorzugsweise werden die Lösemittel ausgewählt aus Ethanol, n- oder i-Propanol, Butanolen, Glycol, Propan- oder Butandiol, Glycerin, Diglyol, Propyl- oder Butyldiglycol, Ketonen wie Aceton und Methylethylketon, Hexylenglycol, Ethylenglycolmethylether, Ethylenglycolethylether, Ethylen-glycolpropylether, Ethylenglycolmono-n-butylether, Diethylenglycol-methylether, Diethylenglycolethylether, Propylenglycolmethyl-, -ethyl- oder -propyl-ether, Butoxy-propoxy-propanol (BPP), Dipropylenglycolmonomethyl-, oder -ethylether, Di-isopropylenglycol-monomethyl-, oder -ethylether, Methoxy-, Ethoxy- oder Butoxytriglycol, 1-Butoxyethoxy-2-propanol, 3-Methyl-3-methoxybutanol, Propylenglycol-t-butylether sowie Mischungen dieser Lösemittel.

Bevorzugte erfindungsgemäße Mittel enthalten ein oder mehrere Lösemittel in einer Gesamtmenge von üblicherweise bis zu 90 Gew.-%, vorzugsweise 0,1 - 30 Gew.-%, insbesondere 2 - 20 Gew.-%, besonders bevorzugt 3 - 15 Gew.-%, äußerst bevorzugt 5 - 12 Gew.-%, z.B. 5,3 oder 10,6 Gew.-%, jeweils bezogen auf das gesamte Mittel.

In einer bevorzugten Ausführungsform enthalten die erfindungsgemäßen Zusammensetzungen mindestens einen weiteren kosmetischen Wirkstoff, der ausgewählt ist aus Monomeren, Oligomeren und Polymeren von Aminosäuren, N-C₂-C₂₄-Acylaminosäuren und/oder den Estern und/oder den physiologisch verträglichen Metallsalzen dieser Substanzen, sowie Mischungen dieser Wirkstoffe.

Die Monomere der Aminosäuren und/oder der N-C₂-C₂₄-Acylaminosäuren sind ausgewählt aus Alanin, Arginin, Asparagin, Asparaginsäure, Canavanin, Citrullin, Cystein, Cystin, Desmosin, Dipalmitoylhydroxyprolin, Glutamin, Glutaminsäure, Glycin, Histidin, Homophenylalanin, Hydroxylysin, Hydroxyprolin, Isodesmosin, Isoleucin, Leucin, Lysin, Methionin, Methylnorleucin, Ornithin, Phenylalanin, Prolin, Pyroglutaminsäure, Sarcosin, Serin, Taurin, Threonin, Thyroxin, Tryptophan, Tyrosin, Valin, N-Acetyl-L-cystein, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat. Besonders bevorzugt sind Lysin, Serin, N-Acetyl-L-cystein, Zink- und Natriumpyroglutamat und Natriumlauroylglutamat.

Der C₂ - C₂₄-Acylrest, mit dem die genannten Aminosäuren an der Aminogruppe derivatisiert sind, ist ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Elaidoyl-, Arachidoyl- oder Behenoyl-Rest. Mischungen von C₈-C₁₈-Acylresten werden auch als Cocoyl-Rest bezeichnet und sind ebenfalls bevorzugte Substituenten. Mit den vorgenannten C₂ - C₂₄-Acylresten können die Aminosäuren, die eine OH-Gruppe tragen, auch an dieser OH-Gruppe verestert sein. Ein erfindungsgemäß bevorzugtes Beispiel hierfür ist Hydroxyprolin, das mit zwei, bevorzugt linearen, C₂-C₂₂-Fettsäureresten N-acyliert und verestert ist, besonders bevorzugt Dipalmitoylhydroxyprolin, das z. B. unter der Bezeichnung Sepilift DPHP von der Firma Seppic erhältlich ist. Die physiologisch verträglichen Salze der erfindungsgemäß bevorzugten Wirkstoffe, die Säuregruppen enthalten und Salze bilden können, sind ausgewählt aus den Ammonium-, Alkalimetall-, Magnesium-, Calcium-, Aluminium-, Zink- und Mangan-Salzen. Bevorzugt sind die Natrium-, Kalium-, Magnesium-, Aluminium-, Zink- und Mangan-Salze.

Unter Aminosäureoligomeren werden erfindungsgemäß Peptide mit 2 - 30, bevorzugt 2 - 15, Aminosäuren, verstanden. Die Oligomere der Aminosäuren und/oder der N-C₂-C₂₄-Acylaminosäuren sind bevorzugt ausgewählt aus Di-, Tri-, Tetra-, Penta-, Hexa- oder Pentadecapeptiden, die N-acyliert und/oder verestert sein können. Zahlreiche dieser Aminosäureoligomere stimulieren die Collagensynthese beziehungsweise sind in der Lage, Zellen des Immunsystems, wie Mastzellen und Makrophagen, zu rekrutieren, die dann über die Freisetzung von Wachstumsfaktoren Reparaturprozesse im Gewebe, z.B. die Collagensynthese, induzieren, bzw. sind in der Lage, an die Sequenz Arg-Phe-Lys in Thrombospondin I (TSP-1) zu binden und damit aktives TGF-β (*tissue growth factor*), der die Synthese von Collagen in dermalen Fibroblasten induziert, freizusetzen. Derartige Aminosäureoligomere können als Wirkstoffe gegen die Hautalterung verwendet werden. Die Polymere der Aminosäuren und/oder der N-C₂-C₂₄-Acylaminosäuren sind bevorzugt ausgewählt aus pflanzlichen und tierischen Proteinhydrolysaten und/oder Proteinen. Tierische Proteinhydrolysate sind z. B. Elastin-, Collagen-, Keratin-, Seiden-, Conchiolin- und Milcheiweiß-Proteinhydrolysate, die auch in Form von Salzen vorliegen können. Erfindungsgemäß bevorzugt sind pflanzliche Proteinhydrolysate, z. B. Soja-, Weizen-, Mandel-, Erbsen-, Kartoffel- und Reisproteinhydrolysate.

In einer weiteren bevorzugten Ausführungsform sind die Polymeren der Aminosäuren ausgewählt aus DNA-Reparaturenzymen. Erfindungsgemäß bevorzugte DNA-Reparaturenzyme sind Photolyase und T4 Endonuclease V, letztere im weiteren mit "T4N5" abgekürzt. Diese beiden Enzyme sind im Stand der Technik bereits als sogenannte DNA-Reparatur-Enzyme bekannt. Unter DNA-Reparatur ist definitionsgemäß die Spaltung bzw. Entfernung von UV-induzierten Pyrimidindimeren aus der DNA zu verstehen.

In einer weiteren bevorzugten Ausführungsform enthalten die erfindungsgemäßen Zusammensetzungen mindestens ein DNA-Oligonucleotid oder mindestens ein RNA-Oligonucleotid.

In einer weiteren bevorzugten Ausführungsform enthalten die erfindungsgemäßen Zusammensetzungen mindestens eine natürliche Betainverbindung. Erfindungsgemäße natürliche Betainverbindungen sind natürlich vorkommende Verbindungen mit der Atomgruppierung R₃N⁺-CH₂-X-COO⁻ gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Betain (Me₃N⁺-CH₂-COO⁻) und Carnitin (Me₃N⁺-CH₂-CHOH-CH₂-COO⁻), jeweils mit Me = Methyl und X = C-C-Einfachbindung (im Falle des Betains) oder X = -CHOH-CH₂- (im Falle des Carnitins).

Erfindungsgemäß besonders bevorzugte Zusammensetzungen sind dadurch gekennzeichnet, dass sie mindestens eine natürliche Betainverbindung in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.

In einer weiteren bevorzugten Ausführungsform enthalten die erfindungsgemäßen Zusammensetzungen mindestens ein Vitamin, Provitamin oder eine als Vitaminvorstufe bezeichnete Verbindung aus den Vitamingruppen A, B, C, E, H und K und den Estern der vorgenannten Substanzen. Vitamin A-palmitat (Retinylpalmitat), Pantolacton, Nicotinsäureamid, Pyridoxin, Pyridoxamin, Pyridoxal, Biotin, Folsäure, Ascorbylpalmitat, Ascorbylacetat, Mg-Ascorbylphosphat, Natriumascorbylphosphat, Natrium- und Magnesiumascorbat und die Tocopherolester, besonders Tocopherylacetat, sind erfindungsgemäß besonders bevorzugt.

In einer weiteren bevorzugten Ausführungsform enthalten die erfindungsgemäßen Zusammensetzungen neben der erfindungsgemäßen Wirkstoffkombination mindestens eine α-Hydroxycarbonsäure, α-Ketocarbonsäure oder β-Hydroxycarbonsäure oder deren Ester-, Lacton- oder Salzform. Erfindungsgemäß bevorzugte α-Hydroxycarbonsäuren oder α-Ketocarbonsäuren sind Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Besonders bevorzugte α-Hydroxycarbonsäuren sind Milchsäure, Citronensäure, Glycolsäure und Gluconsäure. Eine besonders bevorzugte β-Hydroxycarbonsäure ist Salicylsäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Erfindungsgemäß besonders bevorzugte Zusammensetzungen sind dadurch gekennzeichnet, dass sie neben der erfindungsgemäßen Wirkstoffkombination mindestens eine α-Hydroxycarbonsäure, α-Ketocarbonsäure und/oder β-Hydroxycarbonsäure oder ein Derivat, insbesondere einen Ester, ein Lacton oder ein Salz hiervon, in einer Gesamtmenge von 0,01 - 10 Gew.-%, bevorzugt 0,1 - 5 Gew.-%, besonders bevorzugt 0,5 - 1 - 2 Gew.-%, jeweils bezogen auf die gesamte Zusammensetzung, enthalten.

In einer weiteren bevorzugten Ausführungsform enthalten die erfindungsgemäßen Zusammensetzungen mindestens ein Flavonoid oder mindestens einen Flavonoid-reichen Pflanzenextrakt. Erfindungsgemäß außerordentlich bevorzugte Flavonoide sind α-Glucosylrutin, Naringin und Apigenin-7-glucosid. Erfindungsgemäß besonders bevorzugte Zusammensetzungen enthalten mindestens ein Flavonoid in einer Gesamtmenge von 0,0001 bis 1 Gew.-%, bevorzugt 0,0005 bis 0,5 Gew.-% und besonders bevorzugt 0,001 bis 0,1 Gew.-%, jeweils bezogen auf die Flavonoidaktivsubstanz in der gesamten kosmetischen Zusammensetzung.

In einer weiteren bevorzugten Ausführungsform enthalten die erfindungsgemäßen Zusammensetzungen mindestens ein Isoflavonoid oder mindestens einen Isoflavonoid-reichen Pflanzenextrakt. Zu den Isoflavonoiden werden an dieser Stelle die Isoflavone und die Isoflavon-Glycoside gezählt. Erfindungsgemäß besonders bevorzugte Zusammensetzungen sind dadurch gekennzeichnet, dass sie mindestens ein Isoflavonoid in einer Gesamtmenge von 0,00001 bis 1 Gew.-%, bevorzugt 0,0005 bis 0,5 Gew.-% und besonders bevorzugt 0,001 bis 0,1 Gew.-%, jeweils bezogen auf die Isoflavonoidaktivsubstanz in der gesamten kosmetischen Zusammensetzung, enthalten.

In einer weiteren bevorzugten Ausführungsform enthalten die erfindungsgemäßen Zusammensetzungen mindestens ein Polyphenol oder einen Polyphenol-reichen Pflanzenextrakt.

Unter Polyphenolen sind erfindungsgemäß aromatische Verbindungen zu verstehen, die mindestens zwei phenolische Hydroxy-Gruppen im Molekül enthalten. Hierzu zählen die drei Dihydroxybenzole Brenzcatechin, Resorcin und Hydrochinon, weiterhin Phloroglucin, Pyrogallol und Hexahydroxybenzol. In der Natur treten freie und veretherte Polyphenole beispielsweise in Blütenfarbstoffen (Anthocyanidine, Flavone), in Gerbstoffen (Catechine, Tannine), als Flechten- oder Farn-Inhaltsstoffe (Usninsäure, Acylpolyphenole), in Ligninen und als Gallussäure-Derivate auf. Bevorzugte Polyphenole sind Flavone, Catechine, Usninsäure, und als Tannine die Derivate der Gallussäure, Digallussäure und Digalloylgallussäure. Besonders bevorzugte Polyphenole sind die monomeren Catechine, das heißt die Derivate der Flavan-3-ole, und Leukoanthocyanidine, das heißt die Derivate der Leukoanthocyanidine, die bevorzugt in 5,7,3',4',5'-Stellung phenolische Hydroxygruppen tragen, bevorzugt Epicatechin und Epigallocatechin, sowie die daraus durch Selbstkondensation entstehenden Gerbstoffe. Solche Gerbstoffe werden bevorzugt nicht in isolierter Reinsubstanz, sondern als Extrakte gerbstoffreicher Pflanzenteile eingesetzt, z. B. Extrakte von Catechu, Quebracho, Eichenrinde und Pinienrinde sowie anderen Baumrinden, Blättern von Grünem Tee (camellia sinensis) und Mate. Ebenfalls besonders bevorzugt sind die Tannine. Erfindungsgemäß bevorzugt werden die Polyphenole in Mengen von 0,001 bis 10 Gew.-%, besonders bevorzugt 0,005 bis 5 Gew.-% und außerordentlich bevorzugt 0,01 bis 3 Gew.-%, jeweils bezogen auf das Gewicht des Handelsproduktes, das mindestens ein Polyphenol enthält, in der gesamten erfindungsgemäßen Zusammensetzung, eingesetzt.

In einer weiteren bevorzugten Ausführungsform enthalten die erfindungsgemäßen Zusammensetzungen mindestens ein Ubichinon oder ein Ubichinol oder deren Derivate. Ubichinole sind die reduzierte Form der Ubichinone. Die erfindungsgemäß bevorzugten Ubichinone weisen die Formel (UBI-I) auf: 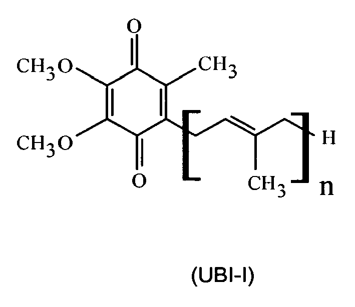 mit n = 6, 7, 8, 9 oder 10.

Besonders bevorzugt ist das Ubichinon der Formel (II) mit n = 10, auch bekannt als Coenzym Q10. Erfindungsgemäß besonders bevorzugte Zusammensetzungen sind dadurch gekennzeichnet, dass sie mindestens ein Ubichinon, Ubichinol oder ein Derivat hiervon in einer Gesamtmenge von 0,0001 - 1 Gew.-%, bevorzugt 0,001 - 0,5 Gew.-% und besonders bevorzugt 0,005 - 0,1 Gew.-%, jeweils bezogen auf die gesamte Zusammensetzung, enthalten.

In einer weiteren bevorzugten Ausführungsform enthalten die erfindungsgemäßen Zusammensetzungen mindestens ein natürlich vorkommendes Xanthin-Derivat, ausgewählt aus Coffein, Theophyllin, Theobromin und Aminophyllin. Erfindungsgemäß bevorzugt sind die natürlich vorkommenden Xanthin-Derivate in Mengen von 0,0001 bis 1 Gew.-%, besonders bevorzugt 0,001 bis 0,5 Gew.-% und außerordentlich bevorzugt 0,005 bis 0,1 Gew.-%, jeweils bezogen auf die gesamte Zusammensetzung, enthalten.

In einer weiteren bevorzugten Ausführungsform enthalten die erfindungsgemäßen Zusammensetzungen Ectoin. Ectoin ist der Trivialname für 2-Methyl-1,4,5,6-tetrahydropyrimidin-4-carboxylat. Erfindungsgemäß bevorzugt ist Ectoin in Mengen von 0,0001 bis 1 Gew.-%, besonders bevorzugt 0,001 bis 0,5 Gew.-% und außerordentlich bevorzugt 0,005 bis 0,01 Gew.-%, jeweils bezogen auf die gesamte Zusammensetzung, enthalten.

In einer weiteren bevorzugten Ausführungsform enthalten die erfindungsgemäßen Zusammensetzungen Kreatin. Kreatin ist der Trivialname für N-Methyl-guanidino-essigsäure bzw. N-Amidinosarkosin. Erfindungsgemäß bevorzugt ist Kreatin in Mengen von 0,0001 bis 1 Gew.-%, besonders bevorzugt 0,001 bis 0,5 Gew.-% und außerordentlich bevorzugt 0,01 bis 0,1 Gew.-%, jeweils bezogen auf die gesamte Zusammensetzung, enthalten.

In einer weiteren bevorzugten Ausführungsform enthalten die erfindungsgemäßen Zusammensetzungen mindestens einen Olivenblattextrakt (Olea Europaea (Olive) Leaf Extract). Olivenblattextrakte können einen hohen Gehalt an Oleanolsäure und/oder Oleanol aufweisen. In einer weiteren bevorzugten Ausführungsform enthalten die erfindungsgemäßen Zusammensetzungen Oleanolsäure, Oleanol und/oder Oleuropein. Erfindungsgemäß besonders bevorzugte Zusammensetzungen sind dadurch gekennzeichnet, dass sie Oleanolsäure, Oleanol und/oder Oleuropein in einer Gesamtmenge von 0,00001 bis 2 Gew.-%, bevorzugt 0,001 bis 1 Gew.-% und besonders bevorzugt 0,05 bis 0,1 Gew.-%, jeweils bezogen auf die gesamte erfindungsgemäße Zusammensetzung, enthalten.

In einer weiteren bevorzugten Ausführungsform enthalten die erfindungsgemäßen Zusammensetzungen Ursolsäure. Erfindungsgemäß besonders bevorzugte Zusammensetzungen sind dadurch gekennzeichnet, dass sie Ursolsäure in einer Gesamtmenge von 0,00001 bis 2 Gew.-%, bevorzugt 0,001 bis 1 Gew.-% und besonders bevorzugt 0,05 bis 0,1 Gew.-%, jeweils bezogen auf die gesamte erfindungsgemäße Zusammensetzung, enthalten.

In einer weiteren bevorzugten Ausführungsform enthalten die erfindungsgemäßen Zusammensetzungen mindestens einen Wirkstoff, der ausgewählt ist aus den Mono- und Polyhydroxystilbenen und deren Estern. Unter Polyhydroxystilbenen werden erfindungsgemäß Stilbene verstanden, die mit 2, 3, 4, 5, 6, 7, 8, 9 oder 10 Hydroxygruppen an den beiden Phenylresten substituiert sind, wobei diese verestert sein können. Mono- und Polyhydroxystilbene und deren Ester erhöhen und/oder verbessern die Interaktion zwischen der extrazellulären Matrix und den Fibroblasten. Erfindungsgemäß besonders bevorzugte Hydroxystilbene und deren Ester sind ausgewählt aus Resveratrol (trans-Stilben-3,4'-5-triol), den Resveratrolmono-, -di- und -triphosphorsäureestern und deren Salzen. Ein erfindungsgemäß besonders bevorzugter Resveratrolphosphorsäureester ist Trisodium Resveratrol Triphosphate, z. B. erhältlich von Ajinomoto.

Erfindungsgemäß besonders bevorzugte kosmetische oder dermatologische Zusammensetzungen sind dadurch gekennzeichnet, dass sie mindestens einen Wirkstoff, der ausgewählt ist aus den Mono- und Polyhydroxystilbenen und deren Estern, in einer Gesamtmenge von 0,000001 - 5 Gew.-%, bevorzugt 0,00001 - 1 Gew.-%, besonders bevorzugt 0,0001 - 0,1 Gew.-% und außerordentlich bevorzugt 0,005 - 0,05 Gew.-%, enthalten, jeweils bezogen auf den Gehalt an Aktivsubstanz in der gesamten Zusammensetzung.

In einer weiteren bevorzugten Ausführungsform enthalten die erfindungsgemäßen Zusammensetzungen mindestens einen sebumregulierenden Wirkstoff. Erfindungsgemäß bevorzugte sebumregulierende Wirkstoffe sind ausgewählt aus 10-Hydroxydecansäure, Sebacinsäure, Azelainsäure und den Estern der Azelainsäure, insbesondere Kaliumazeloyldiglycinat, 1,10-Decandiol und mindestens einem Extrakt aus Spiraea Ulmaria, Glycyrrhizin, das auch als Glycyrrhizinsäure oder Glycyrrhetinsäureglycosid bezeichnet wird und das 2-beta-Glucuronido-alpha-glucuronid der Glycyrrhetinsäure darstellt, sowie deren Salzen, Gerbsäure (tannic acid) und deren Salzen, Gallotanninen, Naringin, Mischungen aus Glycyrrhizin(salzen), Gerbsäure(salzen) und/oder Gallotanninen und Naringin, wie sie zum Beispiel als Handelsprodukt BiSCos Glynarin PF (INCI: Aqua (Water), Alacohol, Ohenoxyethanol, Ammonium Glycyrrhizate, Tannic Acid, Naringine) von der Firma Biesterfeld erhältlich sind, weiterhin aus wasser- und öllöslichen Extrakten aus Hamamelis, Klettenwurzel und Brennessel, Zimtbaumextract (z. B. Sepicontrol^{®} A5 von der Firma Seppic), Chrysanthemenextrakt (z. B. Laricyl^{®} von Laboratoires Sérobiologiques), Hefeproteinhydrolysaten, wie sie z.B. in den Handelsprodukten der Asebiol^{®}-Serie von Laboratoires Serobiologiques erhältlich sind, insbesondere Asebiol^{®} LS 2539 BT 2 (INCl: Aqua, Hydrolyzed Yeast Protein, Pyridoxine, Niacinamide, Glycerin, Panthenol, Allantoin, Biotin) und Asebiol^{®} LS 2539 BT (Aqua, Hydrolyzed Yeast Protein, Pyridoxine, Niacinamide, Glycerin, Panthenol, Propylene Glycol, Allantoin, Disodium Azelate, Biotin) und PEG-8 Isolauryl Thioether, wie es z. B. in dem Handelsprodukt "Antifettfaktor^{®} COS-218/2-A" von Cosmetochem enthalten ist (INCl: Aqua, Cetyl-PCA, PEG-8 Isolauryl Thioether, PCA, Cetyl Alcohol), sowie Mischungen der vorgenannten Substanzen. Bevorzugte Mischungen sind beispielsweise erhältlich als Handelsprodukt Acnacidol PG (Propylene Glycol, 10-Hydroxydecanoic acid, Sebacic acid, 1,10-Decandiol) von Vincience erhältlich. Ein bevorzugter Extrakt aus Spiraea Ulmaria ist z. B. im Produkt Seboregul 2 der Firma Silab enthalten. Kaliumazeloyldiglycinat ist z. B. in dem Produkt Azeloglicina der Firma Sinerga enthalten. Erfindungsgemäß besonders bevorzugte kosmetische oder dermatologische Zusammensetzungen sind dadurch gekennzeichnet, dass sie mindestens einen sebumregulierenden Wirkstoff in Gesamtmengen von 0,00001 bis 10 Gew.-%, bevorzugt 0,01 bis 5 Gew.-% und besonders bevorzugt 0,1 bis 1 -2 Gew.-%, jeweils bezogen auf Aktivsubstanz in der gesamten erfindungsgemäßen Zusammensetzung, enthalten.

Weitere bevorzugte erfindungsgemäße Hautbehandlungsmittel enthalten mindestens einen partikelförmigen, sebumsorbierenden Wirkstoff, insbesondere Talkum, Stärke oder andere Partikel. Erfindungsgemäß bevorzugte kosmetische oder dermatologische Zusammensetzungen sind dadurch gekennzeichnet, dass der partikelförmige, sebumsorbierende Wirkstoff ausgewählt ist aus anorganischen und organischen kosmetischen Adsorbentien mit mittleren Partikeldurchmessern von 0,1 - 100 µm, bevorzugt 0,5 - 50 µm, besonders bevorzugt 5 - 30 µm und außerordentlich bevorzugt 10 - 25 µm. Besonders bevorzugte anorganische Adsorbentien sind ausgewählt aus Kieselsäuren, insbesondere Aerosil^{®}-Typen, Kieselgelen, Siliciumdioxid, Tonen und Schichtsilicaten, insbesondere Bentoniten oder Kaolin, Magnesiumaluminiumsilikaten, insbesondere Talkum, und Bornitrid, sowie Mischungen der genannten Substanzen. Besonders bevorzugte organische Adsorbentien sind ausgewählt aus Stärken und Stärkederivaten, die chemisch und/oder physikalisch modifiziert sein können, insbesondere modifizierten Stärkederivaten vom Typ DRY FLO^{®} der National Starch and Chemical Company, Cellulosepulvern, Lactoglobulinderivaten, insbesondere Natrium-C₈₋₁₆-Isoalkylsuccinyllactoglobulinsulfonat, z. B. als Handelsprodukt Biopol^{®} OE von Brooks Industries erhältlich, Polymerpulvern aus Polyolefinen, Polycarbonaten, Polyurethanen, Polyamiden, Polyestern, Polystyrolen, Polyacrylaten, Polymethacrylaten, Polymethylmethacrylaten, (Meth)acrylat- oder (Meth)acrylat-Vinyliden-Copolymeren, Teflon oder Siliconen, wobei die genannten Polymerpulver in einer besonders bevorzugten Ausführungsform der Erfindung vernetzt sein können, sowie Mischungen der genannten Substanzen.

Polymerpulver auf Basis eines Polymethacrylat-Copolymers sind z. B. als Handelsprodukt Polytrap^{®} 6603 (Dow Corning) erhältlich. Andere Polymerpulver, z. B. auf Basis von Polyamiden, sind unter der Bezeichnung Orgasol^{®} 1002 (Polyamid-6) und Orgasol^{®} 2002 (Polyamid-12) von Elf Atochem erhältlich. Weitere erfindungsgemäß besonders bevorzugte Polymerpulver sind vernetzte Polymethacrylate (Micropearl^{®} M von SEPPIC oder Plastic Powder A von NIKKOL), vernetzte Polymethylmethacrylate (Micropearl^{®} M 305 und Micropearl^{®} M 310 von SEPPIC), Styrol-Divinylbenzol-Copolymere (z. B. Plastic Powder FP von NIKKOL), Polyethylen- und Polypropylen-Pulver (z. B. ACCUREL^{®} EP 400 von AKZO) oder auch Siliconpolymere (z. B. Silicone Powder X2-1605 von Dow Corning).

In einer weiteren bevorzugten Ausführungsform der Erfindung weist der partikelförmige, sebumsorbierende Wirkstoff Hohlräume auf. In einer weiteren bevorzugten Ausführungsform der Erfindung weist der partikelförmige, sebumsorbierende Wirkstoff halbkugelförmige Partikel auf, besonders bevorzugt sind mindestens 50 Gew.-% der Partikel halbkugelförmig. In einer weiteren bevorzugten Ausführungsform der Erfindung weist der partikelförmige, sebumsorbierende Wirkstoff halbkugelförmige Partikel mit Hohlräumen auf, besonders bevorzugt sind mindestens 50 Gew.-% der Partikel halbkugelförmig mit Hohlräumen.

Besonders bevorzugte partikelförmige, sebumsorbierende Wirkstoffe sind vernetzte Polymethylmethacrylate mit mittleren Partikeldurchmessern von 5 - 50 µm, insbesondere die Handelsprodukte Micropearl^{®} M 305 und Micropearl^{®} M 310 von SEPPIC. Außerordentlich bevorzugte partikelförmige, sebumsorbierende Wirkstoffe sind vernetzte Polymethylmethacrylate mit mittleren Partikeldurchmessern von 5 - 50 µm, die halbkugelförmige Partikel mit Hohlräumen umfassen, wobei besonders bevorzugt mindestens 50 Gew.-% der Partikel halbkugelförmig mit Hohlräumen sind, wie insbesondere das Handelsprodukt Micropearl^{®} M 310 von SEPPIC.

Erfindungsgemäß besonders bevorzugte Zusammensetzungen sind dadurch gekennzeichnet, dass sie mindestens einen partikelförmigen sebumsorbierenden Wirkstoff in einer Gesamtmenge von 0,5 - 10 Gew.-%, bevorzugt 1 - 5 Gew.-% und besonders bevorzugt 1,5 - 3 Gew.-%, jeweils bezogen auf die gesamte Zusammensetzung, enthalten.

Weitere erfindungsgemäß beorzugte Hautbehandlungsmittel enthalten mindestens einen adstringierenden Wirkstoff, der zu einer temporären Verengung der Hautporen führt, beispielsweise Tannine bzw. Gerbstoffe bzw. allgemein Polyphenole, weiterhin Aluminiumsalze, wie sie auch als Antitranspirant-Wirkstoffe Verwendung finden. Diese Wirkstoffe regulieren die Sebummenge an der Hautoberfläche durch Verzögerung des Sebumaustritts.

Weitere erfindungsgemäß beorzugte Hautbehandlungsmittel enthalten mindestens einen sebumregulierenden Wirkstoff, der das an der Aktivität der Talgdrüsen beteiligte Enzym 5-alpha-Reduktase inhibiert. Bevorzugte Inhibitoren der 5-alpha-Reduktase sind ausgewählt aus Finasterid^{®}, 4-Androsten-3-on-17β-carbonsäure, (4R)-5,10-seco-19-Norpregna-4,5-dien-3,10,20-trian, (5-alpha,20-R)-4-Diazo-21-hydroxy-20-methylpregnan-3-on, 4-Azasteroide, insbesondere 17β-N,N-diethylcarbamoyl-4-methyl-4-aza-5-alpha-androstan-3-on, 17-alpha-acetoxy-6-methylenpregn-4-en-3,20-dion, 3-Carboxy-androsta-3,5-dien-Steroid-Derivate und 3-Carboxy A Ring-Aryl-Steroide, 3-Androsten-3-carbonsäuren, 3-Carboxy-17β-substituierte Steroide, 17β-(N-t-butylcarboxamid)-5-α-androst-1-en-4-aza-3-on, 17β-(N-t-butylcarboxamid)-androst-3,5-dien-3-carbonsäure, 17β-(N,N-diisopropylcarboxamid)-androst-3,5-dien-3-carbonsäure, 17β-(N,N-diisopropylcarboxamid)-estra-1,3, 5(10)-trien-3-carbonsäure, 17β-(N-t-buty)carboxamid)-estra-1,3,5(10)-trien-3-carbonsäure, 17β-(N,N-diisopropylcarboxamid)-estra-1,3,5(10)-trien-3-sulfonsäure, 17β-(N-t-butylcarboxamid)-estra-1,3,5(10)-trien-3-sulfonsäure, 17β-(N,N-diisopropylcarboxamid)-estra-1,3,5(10)-trien-3-phosphonsäure, 17β-(N-t-butylcarboxamid)-estra-1,3,5(10)-trien-3-phosphonsäure, 17β-(N,N-diisopropylcarboxamid)-estra-1,3,5(10)-trien-3-phosphinsäure, 17β-(N-t-butylcarboxamid)-estra-1,3,5(10)-trien-3-phosphinsäure, 17β-(N-t-butylcarboxamid)-androst-3,5-dien-3-phosphinsäure, 17β-(N,N-diisopropylcarboxamid)-androst-3,5-dien-3-phosphinsäure, 17β-(N-t-butylcarboxamid)-androst-3,5-dien-3-phosphonsäure, (Z)-17β-(N,N-diisopropylcarboxamid)-androst-4-en-3-yliden-essigsäure, 17β-(N,N-diisopropylcarboxamid)-5α-androst-2-en-3-essigsäure, (Z)-17β-(N,N-diisopropylcarboxamid)-5a-androst-3-yliden-essigsäure, 17β-(N,N-diisopropylcarboxamid)-5α-androst-3-en-3-essigsäure und 17β-(N-t-butylcarboxamid)-5a-androst-2-en-3-essigsäure sowie den physiologisch verträglichen Salzen der vorgenannten Säuren.

Erfindungsgemäß besonders bevorzugte Zusammensetzungen sind dadurch gekennzeichnet, dass sie mindestens einen Inhibitor der 5-alpha-Reduktase in einer Gesamtmenge von 0,0001 bis 1 Gew.-%, bevorzugt 0,001 bis 0,5 Gew.-% und besonders bevorzugt 0,01 bis 0,1 Gew.-%, jeweils bezogen auf die gesamte Zusammensetzung, enthalten.

In einer weiteren bevorzugten Ausführungsform enthalten die erfindungsgemäßen Zusammensetzungen mindestens einen organischen hautaufhellenden Wirkstoff. Erfindungsgemäß bevorzugte hautaufhellende Wirkstoffe sind einzeln oder in Mischungen ausgewählt aus Hydrochinon, Kojisäure, Arbutin, Extrakten aus Süßholzwurzel (Glycyrrhiza glabra), Extrakten aus verschiedenen Teilen des Maulbeerbaums (Morus spp., insbesondere aus Morus alba und hier insbesondere Extrakte aus der Baumrinde, dem Stamm, den Blättern und den Früchten), Extrakten aus Scutellaria spp. (Helmkraut), insbesondere aus Scutellaria baicalensis (Baikal-Helmkraut) und hier insbesondere Extrakte aus der Wurzel, Extrakten aus *Waltheria indica* ("Sleepy Morning") und hier insbesondere Extrakte aus den Blättern, Extrakten aus Bärentraube (*Arctostaphylos uva-ursi* (L.), Ericaceae) und hier insbesondere Extrakte aus den Blättern, Extrakten aus Preiselbeere (Blätter und Blüten), Extrakten aus Heidelbeere (Früchte), Extrakten aus Blattknospen von Birnbäumen, Anissamenöl, Extrakten aus Brombeerblättern, weiterhin Extrakten aus *Pyrola rotundifolia* (Rundblättriges Wintergrün), Gurke und/oder Limone, weiterhin Ascorbinsäure, Cumarinsäure ((Z)-2-Hydroxyzimtsäure) und cis-9-Octadecendisäure (andere Nomenklatur: cis-8-Hexadecendicarbonsäure), letztere kommerziell erhältlich z. B. unter der Bezeichnung Arlatone DIOIC DCA von Uniqema, und den Estern und/oder Salzen dieser Säuren. Erfindungsgemäß besonders bevorzugte kosmetische oder dermatologische Zusammensetzungen sind dadurch gekennzeichnet, dass sie mindestens einen organischen hautaufhellenden Wirkstoff in Gesamtmengen von 0,00001 - 10 Gew.-%, bevorzugt 0,01 - 5 Gew.-% und besonders bevorzugt 0,1 - 1 -2 Gew.-%, jeweils bezogen auf Aktivsubstanz in der gesamten erfindungsgemäßen Zusammensetzung, enthalten. Vorteilhafterweise liegen die erfindungsgemäßen Hautbehandlungsmittel in Form einer flüssigen, fließfähigen oder festen Öl-in-Wasser-Emulsion, Wasser-in-Öl-in-Wasser-Emulsion, Makroemulsion, Miniemulsion, Mikroemulsion, PIT-Emulsion, Nanoemulsion, Pickering-Emulsion, Hydrodispersion, eines Hydrogels, einer ein- oder mehrphasigen Lösung oder eines Schaums vor.

In einer besonderen Ausführungsform der erfindungsgemäßen Mittel liegen die Mittel als Mikroemulsion vor. Unter Mikroemulsionen werden im Rahmen der Erfindung neben den thermodynamisch stabilen Mikroemulsionen auch die so genannten "PIT"-Emulsionen verstanden. Bei diesen Emulsionen handelt es sich um Systeme mit den 3 Komponenten Wasser, Öl und Emulgator, die bei Raumtemperatur als Öl-in-Wasser-Emulsion vorliegen. Beim Erwärmen dieser Systeme bilden sich in einem bestimmten Temperaturbereich (als Phaseninversiontemperatur oder "PIT" bezeichnet) Mikroemulsionen aus, die sich bei weiterer Erwärmung in Wasser-in-Ol-Emulsionen umwandeln. Bei anschließendem Abkühlen werden wieder O/W-Emulsionen gebildet, die aber auch bei Raumtemperatur als Mikroemulsionen oder als sehr feinteilige Emulsionen mit einem mittleren Teilchendurchmesser unter 400 nm und insbesondere von etwa 100-300 nm, vorliegen. Erfindungsgemäß können solche Mikro- oder "PIT"-Emulsionen bevorzugt sein, die einen mittleren Teilchendurchmesser von etwa 200 nm aufweisen.

In einer bevorzugten Ausführungsform enthält das erfindungsgemäße Mittel bestimmte Minimalwerte an Parfümöl, nämlich zumindest 0,00001 Gew.-%, vorteilhafterweise zumindest 0,0001 Gew.-%, in beträchtlich vorteilhafter Weise zumindest 0,001 Gew.-%, in vorteilhafterer Weise zumindest 0,01 Gew.-%, in weiter vorteilhafter Weise zumindest 0,1 Gew.-%, in noch weiter vorteilhafter Weise zumindest 0,2 Gew.-%, in sehr vorteilhafter Weise zumindest 0,3 Gew.-%, in besonders vorteilhafter Weise zumindest 0,4 Gew.-%, in ganz besonders vorteilhafter Weise zumindest 0,45 Gew.-%, in erheblich vorteilhafter Weise zumindest 0,5 Gew.-%, in ganz erheblich vorteilhafter Weise zumindest 0,55 Gew.-%, in äußerst vorteilhafter Weise zumindest 0,6 Gew.-%, in höchst vorteilhafterweise zumindest 0,65 Gew.-%, in überaus vorteilhafterweise zumindest 0,7 Gew.-%, in ausnehmend vorteilhafter Weise zumindest 0,75 Gew.-%, in außergewöhnlich vorteilhafter Weise zumindest 0,8 Gew.-%, in außerordentlich vorteilhafter Weise zumindest 0,85 Gew.-%, insbesondere zumindest 0,9 Gew.-% an Parfümöl, bezogen auf das gesamte Mittel. Nach einer weiteren bevorzugten Ausführungsform ist das erfindungsgemäße Mittel frei von Parfümöl-Komponenten, insbesondere in Produkten für sensitive Haut.

Ein weiterer Gegenstand der vorliegenden Erfindung ist die Verwendung von Mischungen aus
a) Dehydroxanthan Gum und
b) mindestens ein vernetztes Copolymer aus Vinylpyrrolidon und 2-Methyl-2[(1-oxo-2-propenyl)amino]-1-propansulfonsäure, das teilweise oder vollständig neutralisiert ist,
c) mindestens einen polymeren Verdicker in Form eines inversen, auto-inversiblen Polymerlatex, enthaltend eine Ölphase, eine Wasserphase, mindestens einen Öl-in-Wasser-Emulgator und mindestens einen verzweigten oder vernetzten Polyelektrolyten, wobei der Polyelektrolyt ausgewählt ist aus
   i) einem Homopolymer, dessen Monomer eine schwache Säurefunktion enthält, die teilweise oder vollständig neutralisiert ist,
   ii) einem Copolymer, das mindestens ein Monomer mit einer starken Säurefunktion sowie ein neutrales Monomer oder ein Monomer mit einer schwachen Säurefunktion enthält.
zur Verdickung wässriger Mittel mit pH-Werten 6, bevorzugt mit pH-Werten im Bereich von 2,5 bis 5,8, außerordentlich bevorzugt 3 bis 5,5, weiter bevorzugt 3,5 bis 5,0, weiter bevorzugt 4,0 bis 4,5.

Bezüglich weiterer bevorzugter Ausführungsformen der erfindungsgemäßen Verwendungen gilt mutatis mutandis das zu den erfindungsgemäßen Mitteln Gesagte.

Die folgenden Beispiele sollen den Gegenstand der Erfindung verdeutlichen, ohne ihn hierauf zu beschränken (alle Mengenangaben in Gew.-%).

| | Nr. 1 | Nr. 2 (Vergleich) | Nr. 3 (Vergleich) | Nr. 4 (Vergleich) |
|---|---|---|---|---|
| AristofleX AVC | 0,7 | 0,7 | - | - |
| Hexandiol-1,6 | 5 | 5 | 5 | 5 |
| Acnacidol BG | 1 | 1 | 1 | 1 |
| Hydrovance | 3 | 3 | 3 | 3 |
| Betain | 2 | 2 | 2 | 2 |
| Natriumsalicylat | 0,5 | 0,5 | 0,5 | 0,5 |
| Natriumbenzoat | 0,5 | 0,5 | 0,5 | 0,5 |
| Zinkgluconat | 0,1 | 0,1 | 0,1 | 0,1 |
| Simulgel EPG | 2 | | 2 | 2 |
| AmazeXT | 2 | 2 | 2 | - |
| Citronensäure-Monohydrat | 0,64 | 0,64 | 0,64 | 0,64 |
| Xanthan Gum | - | - | - | 2 |
| Wasser, vollentsalzt | 82,56 | 84,56 | 83,28 | 83,28 |

Die verdickten kosmetischen Zusammensetzungen Nr. 1 (erfindungsgemäß) und Nr. 2 - 4 (Vergleich) wiesen folgende Viskositäten (20°C, Brookfield-Viskosimeter, TC-Spindel und 4 Umdrehungen pro Minute) auf:

| | Nr. 1 | Nr. 2 (Vergleich) | Nr. 3 (Vergleich) | Nr. 4 (Vergleich) |
|---|---|---|---|---|
| Viskosität | 142.000 mPa·s | 103.000 mPa·s | 92.000 mPa·s | 35.750 mPa·s |

Die erfidungsgemäße Verdickerkombination zeigt eine deutlich synergistische Verdickerleistung gegenüber den Vergleichszusammensetzungen Nr. 2, 3 und 4.

Folgende Rohstoffe wurden verwendet:

| | | |
|---|---|---|
| Aristoflex AVC | Ammonium Acryloyldimethyl taurate/VP Copolymer (92 Gew.-%), t-Butyl Alcohol | Clariant |
| Acnacidol BG | Butylene Glycol, 10-Hydroxydecanoic Acid, Sebacic Acid, 1,10-Decanediol | ISP Vincience |
| Hydrovance | Wasser, N,N'-Bis-(2-hydroxyethyl)harnstoff (45-55 Gew-.%), Harnstoff, Ammoniumlactat | Akzo Nobel |
| Simulgel EPG | Aqua, Sodium Acrylate/Sodium Acryloyldimethyl taurate Copolymer, Polyisobutene, Caprylyl/Capryl Glucoside | Seppic |
| Amaze XT | Dehydroxanthan Gum | Akzo Nobel |

## Patentansprüche

1. Verdickte Zusammensetzung, enthaltend bezogen auf ihr Gewicht
a) 20 bis 95 Gew.-% Wasser und
b) 0,05 bis 5 Gew.-% Dehydroxanthan Gum und
c) mindestens ein vernetztes Copolymer aus Vinylpyrrolidon und 2-Methyl-2[(1-oxo-2-propenyl)amino]-1-propansulfonsäure, das teilweise oder vollständig neutralisiert ist,
d) mindestens einen polymeren Verdicker in Form eines inversen, auto-inversiblen Polymeriatex, enthaltend eine Ölphase, eine Wasserphase, mindestens einen Öl-in-Wasser-Emulgator und mindestens einen verzweigten oder vernetzten Polyelektrolyten, wobei der Polyelektrolyt ausgewählt ist aus
i) einem Homopolymer, dessen Monomer eine schwache Säurefunktion enthält, die teilweise oder vollständig neutralisiert ist,
ii) einem Copolymer, das mindestens ein Monomer mit einer starken Säurefunktion sowie ein neutrales Monomer oder ein Monomer mit einer schwachen Säurefunktion enthält.

2. Zusammensetzung gemäß Anspruch 1, **dadurch gekennzeichnet, dass** sie, bezogen auf ihr Gewicht, 30 bis 94 Gew.-%, vorzugsweise 35 bis 92,5 Gew.-%, besonders bevorzugt 40 bis 90 Gew.-%, weiter bevorzugt 50 bis 87,5 Gew.-% und insbesondere 60 bis 85 Gew.-% Wasser enthält.

3. Zusammensetzung gemäß einem der vorhergehenden Ansprüche, **dadurch gekennzeichnet, dass** sie, bezogen auf ihr Gewicht, 0,1 bis 4 Gew.-%, besonders bevorzugt 0,25 bis 3 Gew.-%, weiter bevorzugt 0,5 bis 2,5 Gew.-% und insbesondere 1 bis 2 Gew.-% Dehydroxanthan Gum b) enthält.

4. Zusammensetzung gemäß einem der vorhergehenden Ansprüche, **dadurch gekennzeichnet, dass** sie, bezogen auf ihr Gewicht, 0,05 bis 3 Gew.-%, vorzugsweise 0,1 bis 2 Gew.-%, besonders bevorzugt 0,2 bis 1,5 Gew.-%, weiter bevorzugt 0,25 bis 1,25 Gew.-% und insbesondere 0,5 bis 1 Gew.-% mindestens eines vernetzten Copolymers c) aus Vinylpyrrolidon und 2-Methyl-2[(1-oxo-2-propenyl)amino]-1-propansulfonsäure, das teilweise oder vollständig neutralisiert ist, enthält.

5. Zusammensetzung gemäß einem der vorhergehenden Ansprüche, **dadurch gekennzeichnet, dass** sie, bezogen auf ihr Gewicht, 0,1 bis 5 Gew.-%, vorzugsweise 0,25 bis 4 Gew.-%, besonders bevorzugt 0,5 bis 3 Gew.-%, weiter bevorzugt 0,75 bis 2,5 Gew.-% und insbesondere 1 bis 2 Gew.-% mindestens eines polymeren Verdickers d) in Form eines inversen, auto-inversiblen Polymerlatex enthält.

6. Zusammensetzung gemäß einem der vorhergehenden Ansprüche, **dadurch gekennzeichnet, dass** die Monomere des Polyelektrolyten im polymeren Verdicker d), die mit einer schwach sauren Gruppe, ausgewählt aus einer -COOH-Gruppe, funktionalisiert sind, ausgewählt sind aus Acrylsäure, Methacrylsäure, Itaconsäure und Maleinsäure, die teilweise oder vollständig neutralisiert sind.

7. Zusammensetzung gemäß einem der vorhergehenden Ansprüche, **dadurch gekennzeichnet, dass** die starke Säurefunktion des Polyelektrolytmonomeren im polymeren Verdicker d) ausgewählt ist aus einer Sulfonsäuregruppe -SO₃H, die teilweise oder vollständig neutralisiert ist.

8. Zusammensetzung gemäß einem der vorhergehenden Ansprüche, **dadurch gekennzeichnet, dass** das Polyelektrolytmonomer mit starker Säurefunktion im polymeren Verdicker d) ausgewählt ist aus 2-Methyl-2[(1-oxo-2-propenyl)amino]-1-propansulfonsäure, die teilweise oder vollständig neutralisiert ist.

9. Zusammensetzung gemäß einem der vorhergehenden Ansprüche, **dadurch gekennzeichnet, dass** die Polyelektrolytmonomere im polymeren Verdicker d), die mit einer schwachen oder starken Säuregruppe funktionalisiert sind, teilweise oder vollständig neutralisiert sind als Natrium-, Kalium-, Ammonium-, Ethanolamin- oder Aminosäure-Salz.

10. Zusammensetzung gemäß einem der vorhergehenden Ansprüche, **dadurch gekennzeichnet, dass** das neutrale Polyelektrolytmonomer im polymeren Verdicker d) ausgewählt ist aus 2-Hydroxyethylacrylat, 2,3-Dihydroxypropylacrylat, 2-Hydroxyethylmethacrylat, 2,3-Dihydroxypropylmethacrylat, den ethoxylierten Derivaten der genannten Ester mit einem Molekulargewicht zwischen 400 und 1000 g/mol, Methylacrylamid, Ethylacrylamid, n-Propylacrylamid, Isopropylacrylamid, Dimethylacrylamid, Diethylacrylamid, Di-n-propylacrylamid, Diisopropylacrylamid sowie Polyvinylpyrrolidon.

11. Zusammensetzung gemäß einem der vorhergehenden Ansprüche, **dadurch gekennzeichnet, dass** sie einen pH-Wert von 1 bis 6,9, vorzugsweise 2 bis 6,5, besonders bevorzugt 2,5 bis 6, außerordentlich bevorzugt 3 bis 5,5, weiter bevorzugt 3,5 bis 5,0, weiter bevorzugt 4,0 bis 4,5 aufweist.

12. Zusammensetzung gemäß einem der vorhergehenden Ansprüche, **dadurch gekennzeichnet, dass** sie als Toilettenreinigungsmittel konfektioniert ist, das zusätzlich mindestens eine organische Säure, ausgewählt aus Citronensäure, Ameisensäure, Essigsäure, Milchsäure, Amidosulfonsäure, Weinsäure, Glycolsäure, Maleinsäure und ihren Mischungen, enthält.

13. Zusammensetzung gemäß einem der vorhergehenden Ansprüche, **dadurch gekennzeichnet, dass** sie als Wäscheweichspulmittel konfektioniert ist, das zusätzlich mindestens ein kationisches Tensid und/oder einen textilwelchmachenden Ton enthält.

14. Verwendung von Mischungen aus
a) Dehydroxanthan Gum und
b) mindestens ein vernetztes Copolymer aus Vinylpyrrolidon und 2-Methyl-2[(1-oxo-2-propenyl)aminol-1-propansulfonsäure, das teilweise oder vollständig neutralisiert ist,
c) mindestens einen polymeren Verdicker in Form eines inversen, auto-inversiblen Polymerlatex, enthaltend eine Ölphase, eine Wasserphase, mindestens einen Öl-in-Wasser-Emulgator und mindestens einen verzweigten oder vernetzten Polyelektrolyten, wobei der Polyelektrolyl ausgewählt ist aus
i) einem Homopolymer, dessen Monomer eine schwache Säurefunktion enthält, die teilweise oder vollständig neutralisiert ist,
ii) einem Copolymer, das mindestens ein Monomer mit einer starken Säurefunktion sowie ein neutrales Monomer oder ein Monomer mit einer schwachen Säurefunktion enthält,
zur Verdickung wässriger Mittel mit pH-Werten < 6.

## Claims

1. Thickened composition, comprising relative to its weight
a) 20 to 95 wt % water and
b) 0.05 to 5 wt % dehydroxanthan gum and
c) at least one crosslinked copolymer of vinyl pyrrolidone and 2-methyl-2[(1-oxo-2-propenyl)amino]-1-propane sulfonic acid that is partially or completely neutralised,
d) at least one polymeric thickener in the form of an inverse, auto-inversible polymer latex, comprising an oil phase, a water phase, at least one oil-in-water emulsifier and at least one branched or crosslinked polyelectrolyte, wherein the polyelectrolyte is selected from
i) a homopolymer, whose monomer comprises a weak acid function that is partially or completely neutralised,
ii) a copolymer that comprises at least one monomer with a strong acid function as well as a neutral monomer or a monomer with a weak acid function.

2. The composition according to claim 1, **characterised in that** it comprises, relative to its weight, 30 to 94 wt %, preferably 35 to 92.5 wt %, particularly preferably 40 to 90 wt %, more preferably 50 to 87.5 wt % and in particular 60 to 85 wt % water.

3. The composition according to one of the previous claims, **characterised in that** it comprises, relative to its weight, 0.1 to 4 wt %, particularly preferably 0.25 to 3 wt %, more preferably 0.5 to 2.5 wt % and in particular 1 to 2 wt % dehydroxanthan gum b).

4. The composition according to one of the previous claims, **characterised in that** it comprises, relative to its weight, 0.05 to 3 wt %, preferably 0.1 to 2 wt %, particularly preferably 0.2 to 1.5 wt %, more preferably 0.25 to 1.25 wt % and in particular 0.5 to 1 wt % of at least one crosslinked copolymer c) of vinyl pyrrolidone and 2-methyl-2[(1-oxo-2-propenyl)amino]-1-propane sulfonic acid that is partially or completely neutralised.

5. The composition according to one of the previous claims, **characterised in that** it comprises, relative to its weight, 0.1 to 5 wt %, preferably 0.25 to 4 wt %, particularly preferably 0.5 to 3 wt %, more preferably 0.75 to 2.5 wt % and in particular 1 to 2 wt % of at least one polymeric thickener d) in the form of an inverse, auto-inversible polymer latex.

6. The composition according to one of the previous claims, **characterised in that** the monomers of the polyelectrolyte in the polymeric thickener d) which are functionalised with a weak acid group, selected from a -COOH group, are selected from acrylic acid, methacrylic acid, itaconic acid and maleic acid, which are partially or completely neutralised.

7. The composition according to one of the previous claims, **characterised in that** the strong acid function of the polyelectrolyte monomers in the polymeric thickener d) is selected from a sulfonic acid group -SO₃H that is partially or completely neutralised.

8. The composition according to one of the previous claims, **characterised in that** the polyelectrolyte monomer with strong acid function in the polymeric thickener d) is selected from 2-methyl-2[(1-oxo-2-propenyl)amino]-1-propane sulfonic acid that is partially or completely neutralised.

9. The composition according to one of the previous claims, **characterised in that** the polyelectrolyte monomers in the polymeric thickener d) which are functionalised with a weak or strong acid group are partially or completely neutralised as the sodium, potassium, ammonium, ethanolamine or amino acid salt.

10. The composition according to one of the previous claims, **characterised in that** the neutral polyelectrolyte monomer in the polymeric thickener d) is selected from 2-hydroxyethyl acrylate, 2,3-dihydroxypropyl acrylate, 2-hydroxyethyl methacrylate, 2,3-dihydroxypropyl methacrylate, the ethoxylated derivatives of the cited esters with a molecular weight between 400 and 1000 g/mol, methylacrylamide, ethylacrylamide, n-propylacrylamide, isopropylacrylamide, dimethylacrylamide, diethylacrylamide, di-n-propylacrylamide, diisopropylacrylamide as well as polyvinyl pyrrolidone.

11. The composition according to one of the previous claims, **characterised in that** it exhibits a pH of 1 to 6.9, preferably 2 to 6.5, particularly preferably 2.5 to 6, exceptionally preferably 3 to 5.5, more preferably 3.5 to 5.0, more preferably 4.0 to 4.5.

12. The composition according to one of the previous claims, **characterised in that** it is made up as a toilet cleaning agent that additionally comprises at least one organic acid, selected from citric acid, formic acid, acetic acid, lactic acid, amido sulfonic acid, tartaric acid, glycolic acid, maleic acid and their mixtures.

13. The composition according to one of the previous claims, **characterised in that** it is made up as a laundry rinse agent that additionally comprises at least one cationic surfactant and/or a fabric-softening clay.

14. Use of mixtures of
a) dehydroxanthan gum and
b) at least one crosslinked copolymer of vinyl pyrrolidone and 2-methyl-2[(1-oxo-2-propenyl)amino]-1-propane sulfonic acid which is partially or completely neutralised,
c) at least one polymeric thickener in the form of an inverse, auto-inversible polymer latex, comprising an oil phase, a water phase, at least one oil-in-water emulsifier and at least one branched or crosslinked polyelectrolyte, wherein the polyelectrolyte is selected from
i) a homopolymer, whose monomer comprises a weak acid function that is partially or completely neutralised,
ii) a copolymer that comprises at least one monomer with a strong acid function as well as a neutral monomer or a monomer with a weak acid function,
for thickening aqueous agents with pH values < 6.

## Revendications

1. Composition épaissie contenant, rapportés à son poids :
a) de 20 à 95 % en poids d'eau ; et
b) de 0,05 à 5 % en poids de gomme de déshydroxanthane ; et
c) au moins un copolymère réticulé de vinylpyrrolidone et de l'acide 2-méthyl-2[(1-oxo-2-propényl)amino]-1-propanesulfonique, qui est neutralisé complètement ou en partie ;
d) au moins un épaississant polymère sous la forme d'un latex polymère inverse autoinversible, contenant une phase huileuse, une phase aqueuse, au moins un émulsifiant du type huile-dans-l'eau et au moins un polyélectrolyte ramifié ou réticulé, le polyélectrolyte étant choisi parmi :
i) un homopolymère dont le monomère contient une fonction d'acide faible, qui est neutralisé en partie ou complètement ;
ii) un copolymère qui contient au moins un monomère possédant une fonction d'acide fort, ainsi qu'un monomère neutre ou un monomère possédant une fonction d'acide faible.

2. Composition selon la revendication 1, **caractérisée en ce qu'**elle contient, rapportés à son poids, de 30 à 94 % en poids, de préférence de 35 à 92,5 % en poids, de manière particulièrement préférée de 40 à 90 % en poids, de manière plus préférée de 50 à 87,5 % en poids, et en particulier de 60 à 85 % en poids, d'eau.

3. Composition selon l'une quelconque des revendications précédentes, **caractérisée en ce qu'**elle contient, rapportés à son poids, de 0,1 à 4 % en poids, de manière particulièrement préférée de 0,25 à 3 % en poids, de manière plus préférée de 0,5 à 2,5 % en poids, et en particulier de 1 à 2 % en poids, de gomme de déshydroxanthane b).

4. Composition selon l'une quelconque des revendications précédentes, **caractérisée en ce qu'**elle contient, rapportés à son poids, de 0,05 à 3 % en poids, de préférence de 0,1 à 2 % en poids, de manière particulièrement préférée de 0,2 à 1,5 % en poids, de manière plus préférée de 0,25 à 1,25 % en poids, et en particulier de 0,5 à 1 % en poids, d'au moins un copolymère réticulé c) de vinylpyrrolidone et d'acide 2-méthyl-2[(1-oxo-2-propényl)amino]-1-propanesulfonique, qui est neutralisé complètement ou en partie.

5. Composition selon l'une quelconque des revendications précédentes, **caractérisée en ce qu'**elle contient, rapportés à son poids, de 0,1 à 5 % en poids, de préférence de 0,25 à 4 % en poids, de manière particulièrement préférée de 0,5 à 3 % en poids, de manière plus préférée de 0,75 à 2,5 % en poids, et en particulier de 1 à 2 % en poids, d'au moins un épaississant polymère d) sous la forme d'un latex polymère inverse autoinversible.

6. Composition selon l'une quelconque des revendications précédentes, **caractérisée en ce que** les monomères du polyélectrolyte dans l'épaississant polymère d), qui sont fonctionnalisés avec un groupe d'acide faible choisi parmi un groupe -COOH-, sont choisis parmi l'acide acrylique, l'acide méthacrylique, l'acide itaconique et l'acide maléique, qui sont en partie ou complètement neutralisés.

7. Composition selon l'une quelconque des revendications précédentes, **caractérisée en ce que** la fonction d'acide fort du monomère du polyélectrolyte dans l'épaississant polymère d) est choisie parmi un groupe d'acide sulfonique -SO₃H qui est en partie ou complètement neutralisé.

8. Composition selon l'une quelconque des revendications précédentes, **caractérisée en ce que** le monomère du polyélectrolyte avec une fonction d'acide fort dans l'épaississant polymère d) est choisi parmi l'acide 2-méthyl-2[(1-oxo-2-propényl)amino]-1-propanesulfonique qui est en partie ou complètement neutralisé.

9. Composition selon l'une quelconque des revendications précédentes, **caractérisée en ce que** les monomères du polyélectrolyte dans l'épaississant polymère d), qui sont fonctionnalisés avec un groupe d'acide faible ou avec un groupe d'acide fort, sont neutralisés en partie ou complètement sous la forme d'un sel de sodium, de potassium, d'ammonium, d'éthanolamine ou d'aminoacide.

10. Composition selon l'une quelconque des revendications précédentes, **caractérisée en ce que** le monomère neutre du polyélectrolyte dans l'épaississant polymère d) est choisi parmi l'acrylate de 2-hydroxyéthyle, l'acrylate de 2,3-dihydroxypropyle, le méthacrylate de 2-hydroxyéthyle, le méthacrylate de 2,3-dihydroxypropyle, les dérivés éthoxylés des esters mentionnés possédant un poids moléculaire entre 400 et 1000 g/mol, le méthylacrylamide, l'éthylacrylamide, le n-propylacrylamide, l'isopropylacrylamide, le diméthylacrylamide, le diéthylacrylamide, le di-n-propylacrylamide, le diisopropylacrylamide et la polyvinylpyrolidone.

11. Composition selon l'une quelconque des revendications précédentes, **caractérisée en ce qu'**elle présente une valeur de pH de 1 à 6,9, de préférence de 2 à 6,5, de manière particulièrement préférée de 2,5 à 6, de manière extraordinairement préférée de 3 à 5,5, de manière plus préférée de 3,5 à 5,0, de manière encore plus préférée de 4,0 à 4,5.

12. Composition selon l'une quelconque des revendications précédentes, **caractérisée en ce qu'**elle est confectionnée sous la forme d'un agent de nettoyage des toilettes, qui contient en outre au moins un acide organique choisi parmi l'acide citrique, l'acide formique, l'acide acétique, l'acide lactique, l'acide amidosulfonique, l'acide tartrique, l'acide glycolique, l'acide maléique, ainsi que leurs mélanges.

13. Composition selon l'une quelconque des revendications précédentes, **caractérisée en ce qu'**elle est confectionnée sous la forme d'un agent adoucissant le linge, qui contient en outre au moins un agent tensioactif cationique et/ou une argile assouplissant les textiles.

14. Utilisation de mélanges
a) de gomme de déshydroxanthane et
b) d'au moins un copolymère réticulé de vinylpyrrolidone et de l'acide 2-méthyl-2[(1-oxo-2-propényl)amino]-1-propane-sulfonique, qui est neutralisé complètement ou en partie ;
c) d'au moins un épaississant polymère sous la forme d'un latex polymère inverse autoinversible, contenant une phase huileuse, une phase aqueuse, au moins un émulsifiant du type huile-dans-l'eau et au moins un polyélectrolyte ramifié ou réticulé, le polyélectrolyte étant choisi parmi :
i) un homopolymère dont le monomère contient une fonction d'acide faible, qui est neutralisé en partie ou complètement ;
ii) un copolymère qui contient au moins un monomère possédant une fonction d'acide fort, ainsi qu'un monomère neutre ou un monomère possédant une fonction d'acide faible ;
pour l'épaississement d'agents aqueux possédant des valeurs de pH < 6.
